(19) Europäisches Patentamt / European Patent Office / Office européen des brevets

(11) **EP 4 260 901 A1**

(12) **EUROPEAN PATENT APPLICATION**

(43) Date of publication:
**18.10.2023 Bulletin 2023/42**

(21) Application number: **23167312.0**

(22) Date of filing: **11.04.2023**

(51) International Patent Classification (IPC):
**A61N 2/00** (2006.01)   **A61N 2/02** (2006.01)

(52) Cooperative Patent Classification (CPC):
**A61N 2/004; A61N 2/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.04.2022 KR 20220046520**

(71) Applicants:
• **Seoul National University R & DB Foundation**
**Seoul 08826 (KR)**

• **Brain & Beyonds Co., Ltd.**
**Seoul 03122 (KR)**

(72) Inventors:
• **PAEK, Sun Ha**
**SEOUL (KR)**
• **CHOI, You Sik**
**SEOUL (KR)**
• **PARK, Soon Beom**
**SEOUL (KR)**

(74) Representative: **Algemeen Octrooi- en Merkenbureau B.V.**
**P.O. Box 645**
**5600 AP Eindhoven (NL)**

(54) **STATIC MAGNETIC FIELD GENERATOR FOR TREATMENT OF CANCER**

(57)   A static magnetic field generator for treatment of cancer according to an embodiment of the present disclosure includes a static magnetic field generating unit, and a space in which a static magnetic field is formed by the static magnetic field generating unit, wherein the formed static magnetic field has a magnitude range of 0.1 mT to 4 T.

[Figure 1]

EP 4 260 901 A1

**Description**

[Technical Field]

The technology disclosed herein relates to a static magnetic field (SMF) generator for treatment of cancer, and more particularly, to a static magnetic field generator that may reduce a size of cancer cells by applying a static magnetic field of a certain size to a treatment region of the body in order to treat diseases, such as tumors and so on of the body.

[Background Art]

Despite the development of modern medicine, cancer is still one of the most incurable diseases among human diseases. Cancer is a tissue made up of abnormal cells and occurs in one or more tissues and spreads to other tissues. More than 100 types of cancer are currently known.
90-95% of main causes of cancer are known to be genetic mutations due to environmental factors, and most of the factors are smoking, obesity, radiation, UV, stress, environmental pollution, and so on.
Cancer has a rapid growth rate and strong ability to invade surrounding tissues, and accordingly, a boundary with normal tissues is not clear, and surgical operation should be performed to remove a tumor and a surrounding tissue as much as possible while greatly reducing functional damage to the body. It is not easy to completely excise the tumor through surgical operation, and there is radiation therapy, an antimetabolic agent, anticancer drug therapy, targeted therapy, immunotherapy, or a combination therapy as an auxiliary treatment method.
However, the known methods may differ for each patient, and generally have side effects, such as hair loss, inhibition of bone marrow function, drug resistance, and organ damage. Numerous efforts have been made worldwide to overcome the side effects and to conquer cancer, but the average survival time of cancer is still short and the possibility of recurrence is high, and accordingly, the development of new treatments is urgently required.
A lot of basic medical research and clinical medical research are still being conducted, and various treatment methods are being proposed, but it is still difficult to cure cancer, and most basic medical research continues to find biological or chemical methods to inhibit a rapid growth and invasion of cancer.
The present disclosure provides a new method that may be applied to cancer treatment by a physical method without using drugs to slow down the growth of cancer cells.

[Disclosure]

[Technical problem]

An object to be achieved by the static magnetic field generator for treatment of cancer, according to an embodiment of the technical idea of the present disclosure, is to provide a static magnetic field generator that generates a static magnetic field showing a therapeutic effect on cancer cells in order to minimize side effects in treating diseases.
An object to be achieved by a static magnetic field generator for treatment of cancer, according to an embodiment of the technical idea of the present disclosure, is not limited to the object to solve the problems described above, and another object not described will be clearly understood by those skilled in the art from descriptions below.

[Technical Solution]

According to an aspect of the present disclosure, a static magnetic field generator for treatment of cancer includes a static magnetic field generating unit, and a space in which a static magnetic field is formed by the static magnetic field generating unit, wherein the formed static magnetic field does not substantially act on normal cells in the space and acts on cancer cells to be killed.
The formed static magnetic field may have a magnitude range of 0.1 mT to 4 T.
According to another aspect of the present disclosure, a static magnetic field generator for treatment of cancer includes a static magnetic field generating unit, and a space in which a static magnetic field is formed by the static magnetic field generating unit, wherein a magnitude of the formed static magnetic field is configured to have a range of 0.1 mT to 4 T.
The static magnetic field may be configured to be generated by a direct current.
The static magnetic field generating unit may have at least one of a solenoid structure, a vertical or horizontal Helmholtz structure, a core coil structure, a multi-stage set coil multi-phase structure, an upper or lower coil selection Helmholtz magnetic field focusing structure, a multi-stage multiple coil multi-phase structure, and a core coil multi-frequency application rotation structure.
The static magnetic field generating unit may include a plurality of coil units, and a space in which the static magnetic field is formed may be placed in a space between the plurality of coil units.

The plurality of coil units may include a first coil unit in which a cylindrical coil is wound multiple times around a first central axis, and a second coil unit which is separated from the first coil by a preset distance and in which a cylindrical coil is wound multiple times around a second central axis coaxial with the first central axis.

Currents of a same magnitude may flow through the first coil unit and the second coil unit in the same direction, and a treatment region of a body may be placed in a space between the first coil unit and the second coil unit.

The first coil unit and the second coil unit may have coils of a same thickness which are wound around a same area in a same number of turns with same inner and outer diameters.

According to another aspect of the present disclosure, the static magnetic field generator for treatment of cancer may further include a first coil housing configured to surround and support the first coil unit, and a second coil housing configured to surround and support the second coil unit.

According to another aspect of the present disclosure, the static magnetic field generator for treatment of cancer may further include a camera configured to image the space in which the static magnetic field is formed.

According to another aspect of the present disclosure, a cancer treatment system includes a bed portion, and the static magnetic field generator defined above and configured to apply a static magnetic field to the bed portion.

According to another aspect of the present disclosure, a static magnetic field generation method for treatment of cancer is configured to generate a static magnetic field in a static magnetic field forming space by using the previously defined static magnetic field generator or the cancer treatment system.

According to another aspect of the present disclosure, a non-transitory computer readable medium includes instructions for causing a processor to perform the method defined above.

According to another aspect of the present disclosure, a static magnetic field generator includes a plurality of coil units configured to generate a static magnetic field for a treatment region of a body, wherein a treatment region of the body is placed in a space between the plurality of coil units.

A magnitude of the generated static magnetic field may be configured to have a range of 0.1 mT to 4 T, and a space in which the static magnetic field may be formed is placed in the space between the plurality of coil units.

The plurality of coil units may include a first coil unit in which a cylindrical coil is wound multiple times around a first central axis, and a second coil unit which is separated from the first coil by a preset distance and in which a cylindrical coil is wound multiple times around a second central axis coaxial with the first central axis.

Currents of a same magnitude may flow through the first coil unit and the second coil unit in the same direction, and a treatment region of a body may be placed in a space between the first coil unit and the second coil unit.

The first coil unit and the second coil unit may have coils of a same thickness which are wound around a same area in a same number of turns with same inner and outer diameters.

[Advantageous Effects]

A static magnetic field generator according to an embodiment of the technical idea of the present disclosure provide a therapeutic effect that reduces sizes of cancer cells by applying only a static magnetic field to a treatment region of the body and reduces side effects.

A static magnetic field generator according to an embodiment of the present disclosure may apply a constant and uniform magnetic field to a treatment region by using a Helmholtz coil and may prevent unexpected side effects by using a low magnetic field harmless to the human body.

In particular, a size of a static magnetic field according to an embodiment of the present disclosure is harmless to the human body, and despite the static magnetic field is very small which corresponds to at least "1 of a thousand or tens of thousands" compared to a size of the discovered static magnetic field of 4 T, the static magnetic field kills cancer cells and significantly reduces invasion of cancer cells, and safety to the human body and cancer treatment efficacy may be ensured at the same time.

In addition, when a static magnetic field of 1.0 mT is applied to tumor cells illustrated in the embodiment of the present disclosure, rather than when a static magnetic field of 0.6 mT is applied, more tumors are killed, and tumor invasion is more significantly reduced, and thus, the larger the magnitude of a static magnetic field, the greater the effect on the tumors.

Furthermore, even when small static magnetic fields of the same size of, for example, 0.6 mT or 1.0 mT is applied to various types of cancer illustrated in the embodiments of the present disclosure, more tumors are killed in proportion to time/date of exposure, and tumor invasion is more significantly reduced, and thus, cancer treatment may be efficiently done by using a very low-level static magnetic field that is harmless to the human body even when applied for a long time.

Effects that may be achieved by a static magnetic field generator according to an embodiment of the technical idea of the present disclosure are not limited to the descriptions above, and other effects not described will clearly understood by those skilled in the art from descriptions below.

[Description of Drawings]

Embodiments will be more clearly understood from the following detailed description taken in conjunction with the accompanying drawings.

FIG. 1 illustrates a schematic diagram of a static magnetic field generator according to an embodiment of the technical idea of the present disclosure.

FIG. 2 illustrates a schematic view of a static magnetic field generator according to an embodiment of the technical idea of the present disclosure, that is a device designed to cover the entire body of a patient.

FIG. 3 is a schematic view of a device for experimenting a treatment effect of a static magnetic field generator according to an embodiment of the technical idea of the present disclosure.

FIGS. 4A and 4B illustrate results of measuring the number of cells in an experimental group (a static magnetic field of 1.0 mT is applied) and a control group (a static magnetic field is not applied) through trypan blue staining for glioblastoma U87-MG.

FIGS. 5A and 5B illustrate results of observing shapes of cells of the experiments of FIGS. 4A and 4B.

FIGS. 6A and 6B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 5% was added.

FIG. 7 is a graph in which cell-covered areas of micrographs of FIGS. 6A and 6B are obtained by using Image J.

FIGS. 8A and 8B are photographs obtained by subdividing at 4-hour intervals the micrographs of FIGS. 6A and 6B from 0 to 28 hours.

FIGS. 9A and 9B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 3% was added.

FIG. 10 is a graph in which cell-covered areas of micrographs of FIGS. 9A and 9B are obtained by using Image J.

FIGS. 11A and 11B are photographs obtained by subdividing at 4-hour intervals the micrographs of FIGS. 9A and 9B from 0 to 28 hours.

FIGS. 12A and 12B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 1% was added.

FIG. 13 is a graph in which cell-covered areas of micrographs of FIGS. 12A and 12B are obtained by using Image J.

FIGS. 14A and 14B are photographs obtained by subdividing at 4-hour intervals the micrographs of FIGS. 12A and 12B from 0 to 28 hours.

FIGS. 15A and 15B are photographs illustrating respective results of live and dead staining before culturing (a control group)/after culturing (an experimental group) in an incubator for 24 hours without applying a static magnetic field, FIG. 15C illustrates a group (the experimental group) obtained by performing the live and dead staining before a static magnetic field is applied for 24 hours, and FIG. 15D illustrates a group (the experimental group) obtained by performing the live and dead staining after a static magnetic field is applied for 24 hours.

FIGS. 16A and 16B illustrate respectively a group (control group) obtained by performing TUNEL staining and a group (experimental group) obtained by performing the TUNEL staining after culturing is performed for 24 hours in an incubator without applying a static magnetic field.

FIGS. 17A and 17B illustrate respectively a group (control group) obtained by performing cell immunochemical staining and a group (experimental group) obtained by performing the cell immunochemical staining after culturing is performed for 24 hours in an incubator without applying a static magnetic field.

FIG. 18 is a photograph illustrating that revelation of Cleaved Caspase-3, Cleaved Caspase-9, and Cleaved PARP increases when a static magnetic field is applied.

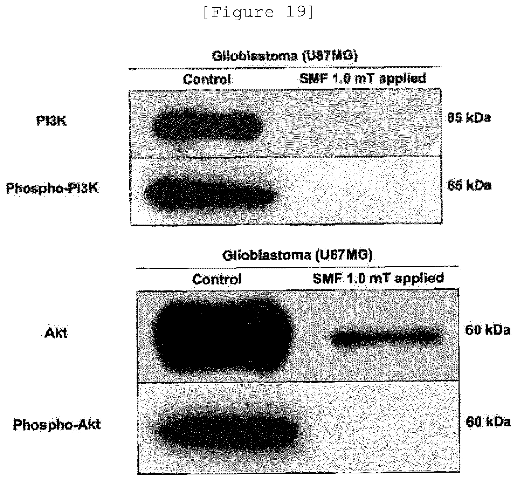

FIG. 19 is a photograph illustrating that both PI3K and PI3K Phosphorylation and Akt and Akt Phosphorylation decrease when a static magnetic field is applied.

FIG. 20 is a photograph illustrating that ferroptosis increases when a static magnetic field is applied.

FIGS. 21A and 21B are photographs illustrating experimental results of shapes of tumor cells when a static magnetic field of 0.6 mT was applied.

FIGS. 22A and 22B are respectively photographs of normal cells (fibroblasts) and graphs illustrating a change in the number of cells before and after a static magnetic field is applied.

FIGS. 23A and 23B illustrate results of measuring the number of cells in an experimental group (a static magnetic field of 0.6 mT is applied) and a control group (a static magnetic field is not applied) through trypan blue staining for glioblastoma cell lines.

FIGS. 24A and 24B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 5% was added.

FIG. 25 is a graph in which cell-covered areas of micrographs of FIGS. 24A and 24B are obtained by using Image J.

FIGS. 26A and 26B are photographs obtained by subdividing at 4-hour intervals the micrographs of FIGS. 24A and 24B from 0 to 28 hours.

FIGS. 27A and 27B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 3% was added.

FIG. 28 is a graph in which cell-covered areas of micrographs of FIGS. 27A and 27B are obtained by using Image J.

FIGS. 29A and 29B are photographs obtained by subdividing at 4-hour intervals the micrographs of FIGS. 27A and 27B from 0 to 28 hours.

FIGS. 30A and 30B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 1% was added.

FIG. 31 is a graph in which cell-covered areas of micrographs of FIGS. 30A and 30B are obtained by using Image J.

FIGS. 32A and 32B are photographs obtained by subdividing at 4-hour intervals the micrographs of FIGS. 30A and 30B from 0 to 28 hours.

FIGS. 33A and 33C illustrate results of measuring the number of cells in an experimental group (a static magnetic field of 1.0 mT is applied) and a control group (a static magnetic field is not applied) through trypan blue staining for a breast cancer cell line MCF-7 and illustrate results of observing shapes of cells.

FIGS. 34A to 34C and FIGS. 35A to 35C illustrate results of measuring the number of cells in an experimental group (a static magnetic field of 1.0 mT is applied) and a control group (a static magnetic field is not applied) through trypan blue staining for a colorectal cancer cell line HCT-116 and illustrate results of observing shapes of cells.

FIGS. 36A to 36C illustrate results of measuring the number of cells in an experimental group (a static magnetic field of 1.0 mT is applied) and a control group (a static magnetic field is not applied) through trypan blue staining for a kidney cancer cell line SNU-333 and illustrate results of observing shapes of cells.

FIGS. 37A to 37C illustrate results of measuring the number of cells in an experimental group (a static magnetic field of 1.0 mT is applied) and a control group (a static magnetic field is not applied) through trypan blue staining for a lung cancer cell line NCI-H1299 and illustrate results of observing shapes of cells.

FIGS. 38A to 38C illustrate results of measuring the number of cells in an experimental group (a static magnetic field of 1.0 mT is applied) and a control group (a static magnetic field is not applied) through trypan blue staining for an ovarian cancer cell line SK-OV-3 and illustrate results of observing shapes of cells.

FIGS. 39A to 39C illustrate results of measuring the number of cells in an experimental group (a static magnetic field of 1.0 mT is applied) and a control group (a static magnetic field is not applied) through trypan blue staining for a pancreatic cancer cell line Capan-2 and illustrate results of observing shapes of cells.

FIGS. 40A to 40C and FIGS. 41A to 41C illustrate results of measuring the number of cells in an experimental group (a static magnetic field of 1.0 mT is applied) and a control group (a static magnetic field is not applied) through trypan blue staining for a prostate cancer cell line DU-145 and illustrate results of observing shapes of cells.

FIGS. 42A to 42C illustrate results of measuring the number of cells in an experimental group (a static magnetic field of 1.0 mT is applied) and a control group (a static magnetic field is not applied) through trypan blue staining for a skin cancer cell line SK-MEL-1 and illustrate results of observing shapes of cells.

FIGS. 43A to 43C illustrate results of measuring the number of cells in an experimental group (a static magnetic field of 1.0 mT is applied) and a control group (a static magnetic field is not applied) through trypan blue staining for a gastric cancer cell line NCI-N87 and illustrate results of observing shapes of cells.

FIGS. 44A to 44C illustrate results of measuring the number of cells in an experimental group (a static magnetic field of 1.0 mT is applied) and a control group (a static magnetic field is not applied) through trypan blue staining for a thyroid cancer cell line SNU-790 and illustrate results of observing shapes of cells.

FIGS. 45 to 47 illustrate various embodiments of a static magnetic field generator applicable to the present disclosure.

[Best Mode]

The technology disclosed in the present disclosure may have various changes and various embodiments, and accordingly, specific embodiments are illustrated in the drawings and described in detail. However, this is not intended to limit the technology disclosed in the present disclosure to specific embodiments, and it should be understood that the technology disclosed herein includes all modifications, equivalents, or substitutes included in the idea and scope of the technology disclosed herein.

In describing the technology disclosed in the present disclosure, when it is determined that detailed descriptions of a related known technology may unnecessarily obscure the subject matter of the technology disclosed in the present disclosure, the detailed descriptions are omitted. In addition, numbers (for example, first, second, and so on) used in the description process of the present disclosure are only identifiers for distinguishing one component from another component.

In addition, in the present disclosure, when one component is referred to as "connected" or "coupled" with another component, the one component may be directly connected or coupled to another component, but unless specifically stated to the contrary, it should be understood that the components may be connected or coupled to each other through another component therebetween.

In addition, in the present disclosure, a component described as "~ portion or unit" may be two or more components combined into one component, or one component may be divided into two or more for each more subdivided function. In addition, each component to be described below may additionally perform some or all of functions of another component in addition to a main function thereof, and some of the main function of each component may be exclusively performed by another component.

Expressions, such as "first" and "second" used in various embodiments, may modify various components regardless of order and/or importance and do not limit the corresponding components. For example, a first component may be referred to as a second component, and similarly, the second component may also be referred to as the first component without departing from the scope of the technology disclosed herein.

In the present disclosure, in order to check cancer cell removal and inhibition effects according to the technical idea of the present disclosure that applies a static magnetic field to cancer cells to remove and/or inhibit the cancer cells, a result of checking what kind of change occurs when weak static magnetic fields of 0.6 mT and 1.0 mT are applied to a glioblastoma cell line U87-MG is provided. This is only an example, and in particular, the technical idea of the present disclosure is applicable to all types of static magnetic field generators capable of generating density of a magnetic flux in a range of 0.1 mT to 4 T, and the magnitude of the static magnetic field is lower 30,000 times than the magnetic flux density of 3 T used in a general magnetic resonance imaging device, and accordingly, it should be noted that the magnitude of the static magnetic field is safe for the human body even when being continuously used over several days for, for example, treatment.

In addition, although glioblastoma is described as an example in the present disclosure, the technical idea of the present disclosure is not limited thereto and it will be understood that the technical idea of the present disclosure corresponds to a treatment device and a treatment method that may be applied to all types of cancer cells.

## Static Magnetic Field Generator

Hereinafter, a static magnetic field generator according to a preferred embodiment will be described in detail. In addition, in the present disclosure, a size of a static magnetic field may be adjusted according to a size of a current by using a coil structure, a static magnetic field generator having a structure of a Helmholtz coil may be described as an example to obtain a uniform magnetic field, and for example, a specific magnet with a fixed magnetic flux density or a special magnetic field generating device may be used as the magnetic field generator.

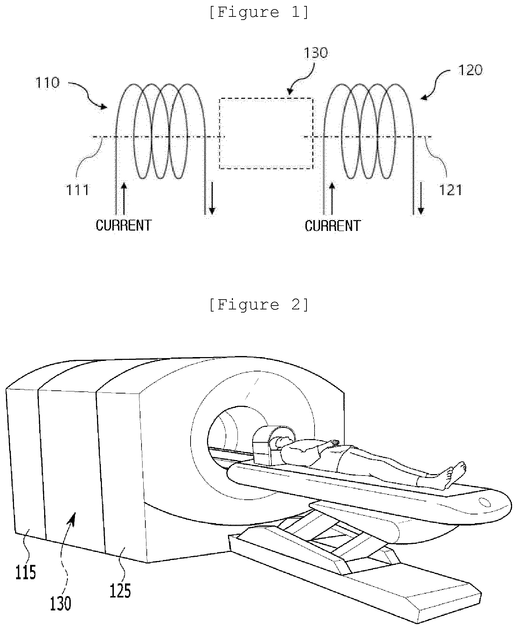

FIG. 1 illustrates a schematic view of a coil of a static magnetic field generator according to an embodiment of the technical idea of the present disclosure. FIG. 2 exemplarily illustrates a schematic view of a static magnetic field generator according to an embodiment of the technical idea of the present disclosure, that is a device designed to cover the entire body of a patient.

The static magnetic field generator according to the present embodiment may include a first coil unit 110, a second coil unit 120, a treatment space 130, and a camera. Here, the first coil unit 110 and the second coil unit 120 each have a Helmholtz coil structure.

The first coil unit 110 may have a cylindrical coil wound multiple times around a first central axis 111.

The second coil unit 120 may be separated from the first coil unit 110 by a preset distance and may have a cylindrical coil wound multiple times around a second central axis 121 coaxial with the first central axis 111. Here, a space separated by a preset distance becomes the treatment space 130 where a treatment region (a treatment portion) of the body is located. The treatment space 130 becomes a region where a uniform magnetic field is formed. The treatment space 130 is not limited in size, and in particular, may have a form of a very large space (for example, a chamber, a room, or so on) that may provide continuous treatment for several hours to several days due to very low magnetic flux density, that is, a magnetic field size in the present embodiment.

The first coil unit 110 and the second coil unit 120 may have coils of the same thickness wound around the same area in the same number of turns with the same inner and outer diameters. An average diameter of the first coil unit 110 and the second coil unit 120 may be determined as an intermediate value of the inner diameter and the outer diameter.

A current of the same magnitude and in the same direction may flow through the first coil unit 110 and the second coil unit 120, a magnetic field to treat a treatment region of the body is uniformly formed in the treatment space 130 between the first coil unit 110 and the second coil unit 120, and magnetic flux density, which is the magnitude of a magnetic field to be formed, ranges from 0.1 mT to 4 T. 0.1 mT is a minimum size of a static magnetic field required for removal and inhibition of cancer cells, and when the size of the static magnetic field exceeds 4 T, the human body may be harmed. In the present embodiment, for example, the magnitude of a magnetic flux density of 0.1 mT corresponds to a very weak static magnetic field that is "30,000 times" lower because the magnetic flux density used by a magnetic resonance imaging device is 3 T. Although a static magnetic field of 1 mT which is another exemplary size is harmless to the human body and has very small magnitude of about "1/4,000" compared to the magnitude of a static magnetic field of 4 T which is an upper limit of the discovered magnitude, tumors are killed and tumor invasion is significantly reduced, and even under continuous treatment, safety to the human body and cancer treatment efficacy may be ensured at the same time.

In order for the first coil unit 110 and the second coil unit 120 to configure a specific treatment device, a first coil housing 115 supporting the first coil unit 110 and a second coil housing 125a supporting the second coil unit 120 may be formed, and a member that covers the treatment space 130 and connects the first coil housing 115 to the second coil housing 125 may be placed between the first coil housing 115 and the second coil housing 125. According to the example of FIG. 2, a patient may be slid into the treatment space 130 through the first coil unit 110 or the second coil unit 120. A distance between the first coil unit 110 and the second coil unit 120 may be adjusted to apply a static magnetic field to the entire body of a patient.

In order to monitor conditions of the patient receiving treatment in the treatment space 130, a camera may be installed to capture an image of the treatment space 130, and an image captured by the camera may be transmitted to a server through a wired or wireless network.

In addition, a treatment method using a static magnetic field, according to an embodiment of the present disclosure, may be performed by following steps.

First, a step of placing a treatment region of the body in the treatment space 130 may be performed. For example, the treatment space 130 may be placed between the first coil unit 110 and the second coil unit 120. Here, the first coil unit 110 has a cylindrical coil wound multiple times around the first central axis 111, and the second coil unit 120 is separated from the first coil unit 110 by a preset distance and has a cylindrical coil wound multiple times around the second central axis 121 coaxial with the first central axis 111. In addition, the first coil unit 110 and the second coil unit 120 may be set to have coils of the same thickness wound around the same area in the same number of turns with the same inner and outer diameters, but are not limited thereto.

Next, a step of applying a current having the same magnitude to the first coil unit 110 and the second coil unit 120 in the same direction may be performed. It is preferable that the first coil unit 110 and the second coil unit 120 form a static magnetic field having magnetic flux density (a size of a static magnetic field) of 0.1 mT to 4 T.

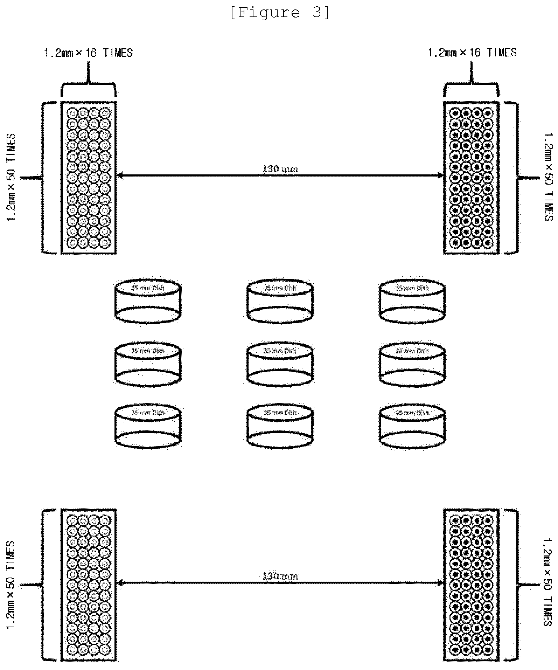

As described above, the structure of the Helmholtz coil made according to an embodiment of the present disclosure is illustrated in FIG. 3 in order to verify efficacy of a static magnetic field of a uniform size on cancer cells. FIG. 3 is a schematic view of a static magnetic field generator according to an embodiment of the present disclosure.

As illustrated exemplarily in FIG. 3, a diameter of an enameled copper wire used at the time winding a coil is 1.2 mm, and the number of turns of the enamel copper wires is 16 in a horizontal direction and 50 in a vertical direction, the total number of winding is 800.

An inner diameter thereof is designed to be 130 mm by considering a margin to accommodate several cell culture dishes, each having an inner diameter of 35 mm. An outer diameter is 168.4 mm because the enameled copper wire is wound 16 times in the horizontal direction along the length of the inner diameter. Since the coil is wound several times, a diameter used when designing the coil corresponds to an average diameter. An average diameter is 149.2 mm which is obtained by summing an inner diameter and an outer diameter and dividing the summed value by 2, and an average radius is 74.6 mm which is obtained by dividing the average diameter by 2 (D = 149.2 mm and R = 74.6 mm).

A current I, which is supplied such that the magnitude of magnetic flux density is, for example, 1.0 mT, is 103.06 mA which is obtained by using the Bio-Savar law from the Helmholtz coil structure.

Changes caused by the static current magnetic field generator were checked from cell proliferation by directly measuring the number of cells through trypan blue staining, and the number of cells and the shape of cells were observed through microscopic observation. In addition, cell live and dead were confirmed through Calcein-AM staining, and cell apoptosis was confirmed through TUNEL staining.

## Brain Cancer Cell Line (Glioblastoma) and Culture Method

Human glioblastoma cell line U87-MG, which is a brain cancer used in the example, was purchased from American type culture collection (ATCC) (Manassas, VA, USA). Dulbecco's modified eagle's medium (DMEM) (WELGENE, Korea) badge to which fetal bovine serum (FBS) (Gibco Corporation, Grand Island, NY, USA) of 10% and antibiotic-antimycotic (AA) (Gibco Corporation, Grand Island, NY, USA) of 1% were added, was used for cell culture.

U87-MG cells were cultured in an incubator of 37°C and 5% CO2, and observed every day, and it was considered confluence when filled by 70 to 80% of an area of a cell culture dish of 100 mm, and subculture was performed by using TryPLE (Gibco Corporation, Grand Island, NY, USA). In addition, the badge was replaced with a new badge every 2 to 3 days.

In the cell live and dead observation experiment, the glioblastoma cell line U87-MG was seeded in a cell culture dish of 35 mm and cultured in an incubator of 37°C and 5% CO2 for 24 hours. After 24 hours and 48 hours, live and dead staining were performed.

In the present embodiment, when a static magnetic field is applied, cells are planted, and then, the cells were cultured in an incubator of 37°C and 5% CO2 without applying the static magnetic field for 24 hours. Thereafter, the static magnetic field was continuously applied for 24 hours, and on the next day, application of the static magnetic field was stopped to end the experiment.

In the present embodiment, LIVE/DEAD™ cell Imaging kit (Invitrogen, Carlsbad, CA, USA) was used for a cell live and dead staining experiment, and a protocol provided by a manufacturer was used as an experimental protocol. Before cell live and dead staining is performed, washing is done with 1x PBS, and then, a LIVE/DEAD™ staining experiment was performed. After the cells are wrapped with foil to prevent reaction from occurring due to external light, the cells were placed in an incubator for 30 minutes. Thereafter, live and dead cells were observed by using a fluorescence microscope to observe a change in glioblastoma cell line U87-MG due to a static magnetic field.

### Change in Tumor Cells in Static Magnetic Field: Cell Proliferation and Cell Migration

When the glioblastoma cell line U87-MG was densely packed in 70 to 80% of a bottom area of the cell culture dish of 100 mm, a cell proliferation measurement experiment was started. On the day of the experiment, $1 \times 10^4$ cells were planted in a cell culture dish of 35 mm. Thereafter, the number of live cells unstained through trypan blue staining (trypan blue exclusion) was counted every 24 hours.

In the present embodiment, when a static magnetic field is applied, a static magnetic field was applied after about 12 hours after the cells were planted, and the static magnetic field was continuously applied for 72 hours after the experiment. The reason is that, when the static magnetic field is applied shortly after the cells are planted, it is not possible to check whether the cells are changed by the static magnetic field or there is a change because the cells are in poor condition, and thus, the present disclosure prevents this.

In the cell migration observation experiment, the time when the glioblastoma cell line U87-MG grew by 80 to 90% of an area of the cell culture dish of 35 mm was regarded as a dense state. Then, a scratch experiment (scratch assay) was performed in which cells on the cell culture dish were scratched in a vertical direction from top to bottom by using a pipette tip of 100 uL. After removing the scratched cells and washing is done with 1x phosphate buffered saline (PBS) to remove the existing culture fluid remaining in the cells as much as possible, newly added culture fluids had different FBS concentrations of 10%, 5%, 3%, and 1%. The reason is to minimize effects of FBS and observe only cell migration because the FBS includes an abundance of growth factors and may be affected by cell differentiation, cell proliferation, and so on. The cells were continuously imaged at 1 minute intervals for 72 hours by using a microscope equipped with a real-time cell monitoring device (live cell imaging platform).

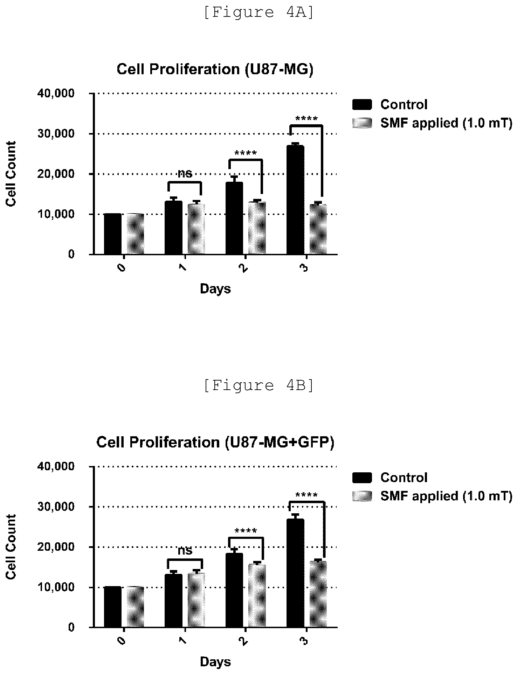

FIGS. 4A and 4B illustrate results of measuring the number of cells in an experimental group (a static magnetic field of 1.0 mT is applied) and a control group (a static magnetic field is not applied) through trypan blue staining for glioblastoma U87-MG. In order to investigate an effect of a static magnetic field on cell proliferation in U87-MG cell which is a glioblastoma cell line and U87-MG+GFP cell which is transfected to produce green fluorescent protein (GFP), the number of cells was directly counted through trypan blue staining (trypan blue exclusion), and at the same time, a shape of the cell was also observed through a microscope.

The same number of $1 \times 10^4$ cells were planted in a culture dish of 35 mm at the beginning of an experiment for both an experimental group to which a static magnetic field was applied and a control group to which no static magnetic field was applied. The static magnetic field started to be applied after the cells were planted and then about 12 hours elapsed, and the static magnetic field was continuously applied for 72 hours until the experiment was finished.

The number of U87-MG cells in the control group and the number of U87-MG cells in the experimental group were respectively $1.31 \times 10^4$ and $1.24 \times 10^4$ after 24 hours from when the experiment starts, and the number of U87-MG cells in the control group and the number of U87-MG cells in the experimental group were respectively $1.78 \times 10^4$ and $1.29 \times 10^4$ after 48 hours from when the experiment starts, and a difference between the two groups began to appear from this time. Finally, after 72 hours from when the experiment started, the number of cells in the control group and the number of cells in the experimental group was respectively $2.69 \times 10^4$ and $1.23 \times 10^4$, and the difference therebetween was further increased.

The number of U87-MG+GFP cells in a control group and the number of U87-MG+GFP cells in an experimental group were respectively $1.31 \times 104$ and $1.33 \times 104$ after 24 hours from when the experiment started, and the number of U87-MG+GFP cells in a control group and the number of U87-MG+GFP cells in an experimental group were respectively $1.83 \times 104$ and $1.55 \times 104$ after 48 hours from when the experiment started, a difference between the two groups began to appear from this time. Finally, after 72 hours from when the experiment started, the number of cells in the control group and the number of cells in the experimental group was respectively $2.68 \times 104$ and $1.63 \times 104$, and the difference therebetween was further increased.

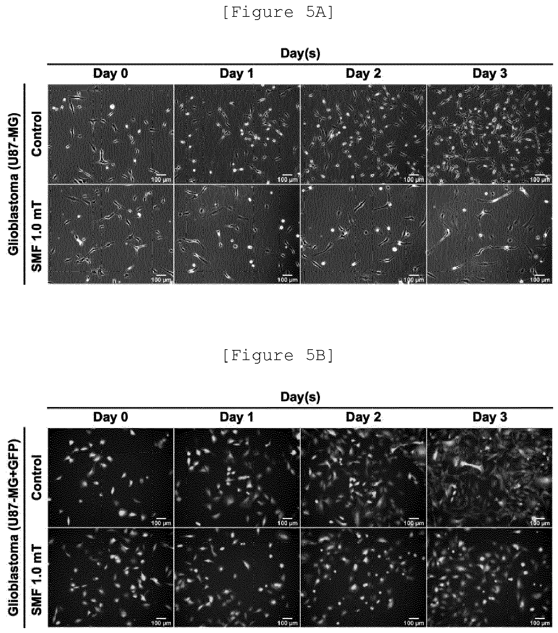

FIGS. 5A and 5B illustrate results of observing shapes of cells of the experiments of FIGS. 4A and 4B.

U87-MG cells in the experimental group increased at an average rate of $0.07 \times 104$ cells per day, U87-MG cells in the control group increased at an average rate of $0.56 \times 104$ cells per day, U87-MG+GFP cells in the experimental group increased at an average rate of $0.21 \times 104$ cells per day, and U87-MG+GFP cells in the control group increased at an average rate of $0.56 \times 104$ cells per day. Therefore, in the present embodiment, it was confirmed that a static magnetic field of 1.0 mT acts to prevent cell proliferation of U87-MG cells and U87-MG+GFP cells, which are glioblastoma cell lines. In addition, in the present embodiment, it was confirmed from the experimental results of U87-MG cells and U87-MG+GFP cells that effects of static magnetic fields of the same magnitude of 1.0 mT are different from each other depending on cells.

As may be seen from the same drawings, cell proliferation occurs very well in a control condition where no magnetic field is applied, and the cells try to fill all regions observed by the microscope, and in contrast to this, in the present embodiment, cell proliferation is reduced in a condition of SMF 1.0 mT to which a static magnetic field is applied, and cells does not fill the region compared to photographs of a microscope in the control condition, and the number of round shapes increases. That is, during cell division, the number of cells had to be increased and sizes of the cells had to be increased, but actually, the number of cells was reduced and the sizes of the cells were reduced, and there were many cells having round shapes.

In order to examine effects of a static magnetic field on cell migration of a glioblastoma cell line U87-MG, cells were continuously observed for 3 days by using a live cell imaging system.

After U87-MG cells are planted, a glioblastoma cell line, in a 35 mm cell culture dish, the culture fluid with 10% FBS added was used until the density reached 70 to 80%. At the beginning of the experiment, the concentration of FBS added to the culture fluid was changed. Migration of cells was observed in real time at different concentrations of 5%, 3% and 1%. After a U87-MG cell of a glioblastoma cell line was planted in a cell culture dish of 35 mm, the culture fluid to which FBS of 10% has been added was used until 70 to 80%, and at the beginning of the experiment, the concentrations of FBS added to the culture fluid were differently set to 5%, 3%, and 1%, and migration of cells was observed in real time. The reason why the experiment was performed with different concentrations of FBS is that FBS includes not only many growth factors necessary for cell growth but also various types of protein and so on, and thus, survival, growth, and division of cells are greatly affected.

That is, in order to perform a cell migration analysis method, quantification was performed to obtain an area surrounded by cells in a region of interest (RoI) with frames at 0 hour, 12 hours, 24 hours, 48 hours, and 72 hours by using software. After obtaining the total area of one frame by using Image J, an area of an empty part, that is, an area not filled with cells, was obtained. In addition, by subtracting these two, the area surrounded by the cells was obtained, and a ratio occupied by cells in one frame was named a cell-covered area and illustrated in a graph.

[Equation 1]

$$Cell-Covered\,Area\,[\%] = \frac{total\,area\,of\,one\,frame\,-\,empty\,area}{total\,area\,of\,one\,frame} \times 100\,[\%]$$

The experimental results may be checked through related drawings.

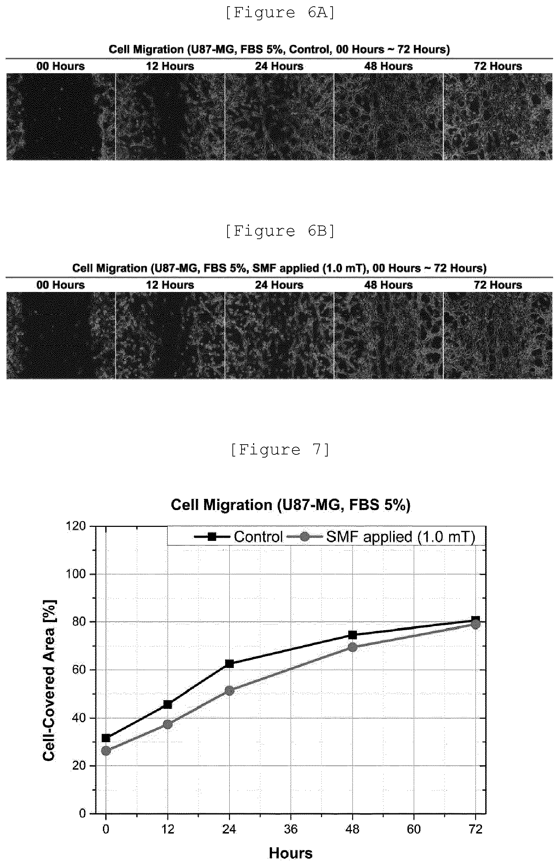

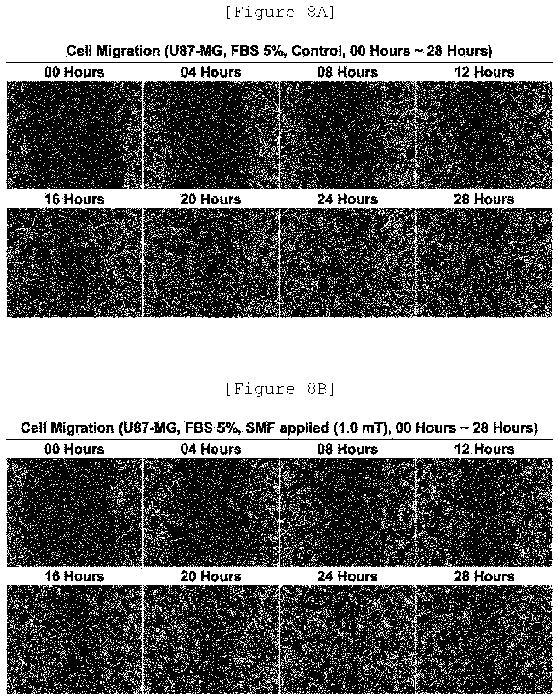

FIGS. 6A and 6B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 5% was added. FIG. 7 is a graph in which cell-covered areas of micrographs of FIGS. 6A and 6B are obtained by using Image J. FIGS. 8A and 8B are photographs obtained by subdividing at 4-hour intervals the micrographs of FIGS. 6A and 6B from 0 to 28 hours. This is because a point at which migration of cells begins to be inhibited appears at the beginning of an experiment (for example, between 0 and 28 hours).

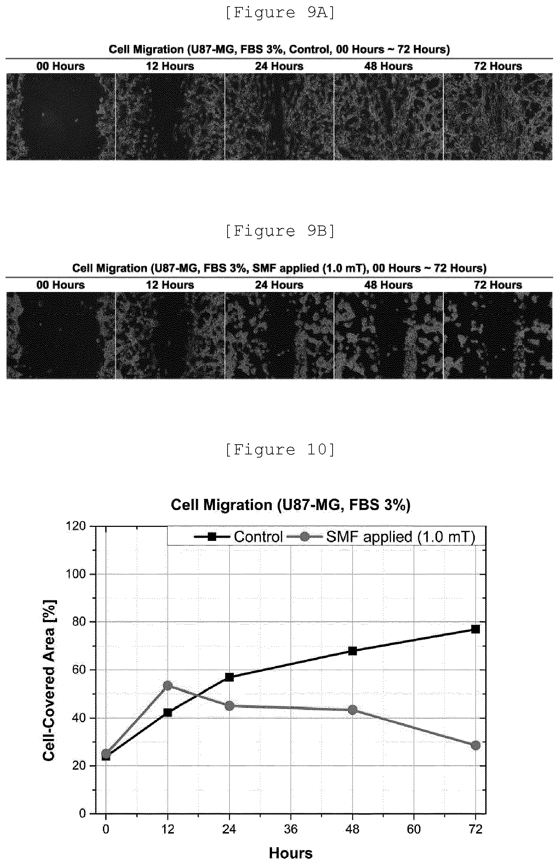

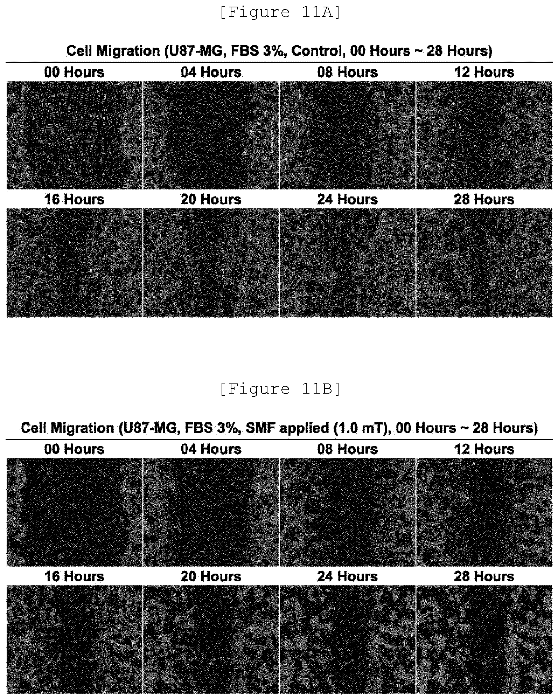

FIGS. 9A and 9B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 3% was added. FIG. 10 is a graph in which cell-covered areas of micrographs of FIGS. 9A and 9B are obtained by using Image J. FIGS. 11A and 11B are photographs obtained by subdividing at 4-hour intervals the micrographs of FIGS. 9A and 9B from 0 to 28 hours. As may be seen from the same drawings, first, when a magnetic field is not applied, it may be observed that cells migrate over time toward the center in which there are no cells. In addition, it may be seen that, when a magnetic field is applied, the cells migrate over time toward the center in which there are no cells until 16 hours, and then, shapes of the cells begin to change after 20 hours and the cells stop migration.

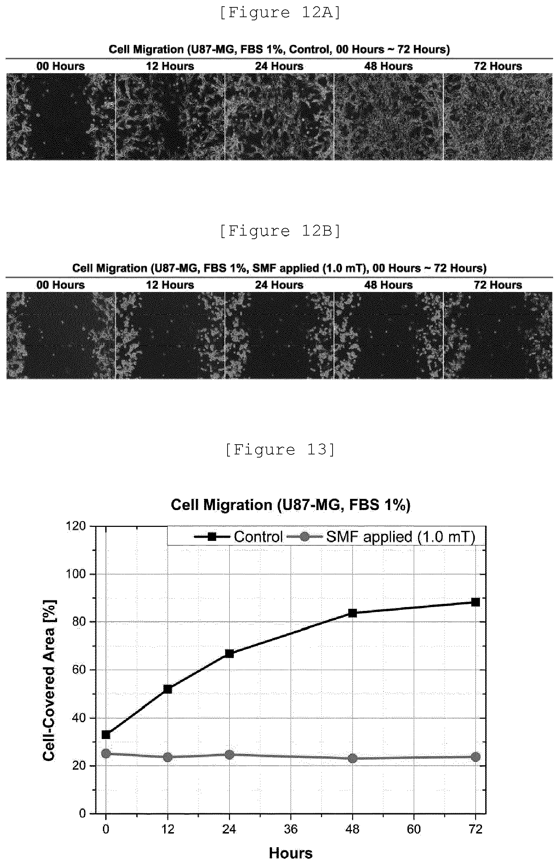

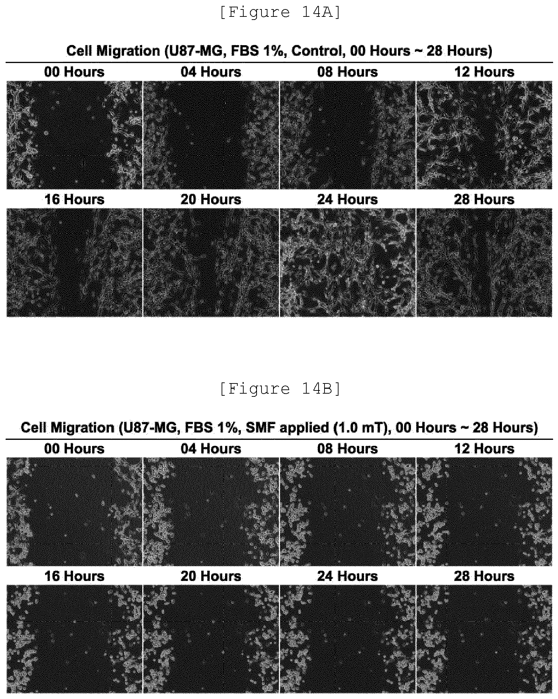

FIGS. 12A and 12B illustrate photographs taken under a microscope of an experimental group (a static magnetic field of 1.0 mT is applied) and a control group (a static magnetic field is not applied) when 0 hour, 24 hours, 48 hours, and 72 hours elapsed after a cell migration experiment of glioblastoma U87-MG was performed with a culture fluid to which FBS of 1% was added. FIG. 13 is a graph in which cell-covered areas of micrographs of FIGS. 12A and 12B are obtained by using Image J. FIGS. 14A and 14B are photographs obtained by subdividing at 4-hour intervals the micrographs of FIGS. 12A and 12B from 0 to 28 hours.

**Change in Tumor Cells in Static Magnetic Field: Cell Fluorescence Staining**

Another method used to check whether the glioblastoma cell line U87-MG is affected by a static magnetic field is cell live and dead staining, cell dead staining (TUNEL), and cell immunochemical staining (Ki-67). When a cell staining experiment was performed, the added concentration of FBS was 3%. The glioblastoma cell line U87-MG was planted in a cell culture dish of 35 mm, and the, cultured in an incubator of 37°C and 5% CO2 for 48 hours. Staining was performed after 24 hours from when cells were planted, and another staining was performed after 48 hours from when the cells were planted.

In a method of applying a static magnetic field according to the present embodiment, the static magnetic field was not applied for 24 hours after cells were planted, and the cells were cultured in an incubator of 37°C and 5% CO2. Thereafter, the static magnetic field was continuously applied for 24 hours, and then, the application was stopped, and the cells were stained. That is, staining was performed before the static magnetic field was applied and after the static magnetic field was applied for 24 hours.

LIVE/DEAD™ cell imaging kit (Invitrogen, Carlsbad, CA, United States) was used for a cell live and dead staining experiment, and a protocol provided by a manufacturer was used as an experimental protocol thereof. Before cell live and dead staining is performed, washing was done with 1xPBS, and then, a LIVE/DEAD™ staining experiment was performed. In addition, the cell was wrapped with aluminum foil to prevent the cell from reacting on external light, and then, the cell was reacted in an incubator of 37°C and 5% CO2 for 30 minutes. Thereafter, live and dead cells were observed by using a fluorescence microscope. In addition, terminal deoxynucleotidyl transferase dUTP Nick-End labeling (TUNEL) staining was performed to check DNA fragmentation, which is one of characteristics of cell dead. Cells were prepared in the same way as in the cell live and dead staining experiment, and a DeadEnd™ fluorometric TUNEL system (Promega, Madison, WI, United States) product was used, and a protocol provided by a manufacturer was used as an experimental protocol. Finally, Ki-67, that was a cell proliferation marker, was examined to check there was a change when a static magnetic field was applied and when the static magnetic field was not applied through cell immunocytochemistry (ICC). A monoclonal antibody Ki-67 Antigen (MIB-1) (1:200) (Agilent, Carpinteria, CA, United States) was used as a primary antibody used in the present example, and Alexa Fluor® Plus 594 (1:200) (Invitrogen, Carlsbad, CA, United States) was used as a secondary antibody.

Calcein-AM staining was performed to examine an effect of a static magnetic field on cell live and dead of a glioblastoma cell line U87-MG. Since Calcein-AM is cell-permeable staining, the Calcein-AM selectively penetrates only live cells and stains to green fluorescence, and BOBO-3 Iodide couples to DNA of dead cells and illustrates red fluorescence, and accordingly, live cells may be easily distinguished from dead cells at the same time.

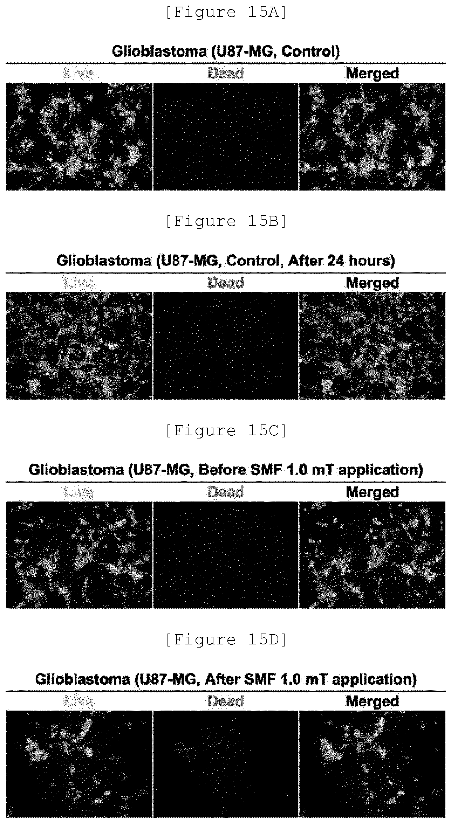

When comparing results of staining of a LIVE/DEAD™ cell imaging kit before and after a static magnetic field is applied, red fluorescence-stained portions appeared only in the cells to which the static magnetic field was applied. FIGS. 15A to 15D illustrate results of the staining of the LIVE/DEAD™ cell imaging kit, and FIGS. 15A and 15B illustrate respectively groups (control groups) obtained by performing live and dead staining before and after culturing was performed in an incubator for 24 hours without applying a static magnetic field, and FIGS. 15C and 15D illustrate respectively groups (control groups) obtained by performing live and dead staining before and after the static magnetic field was applied for 24 hours.

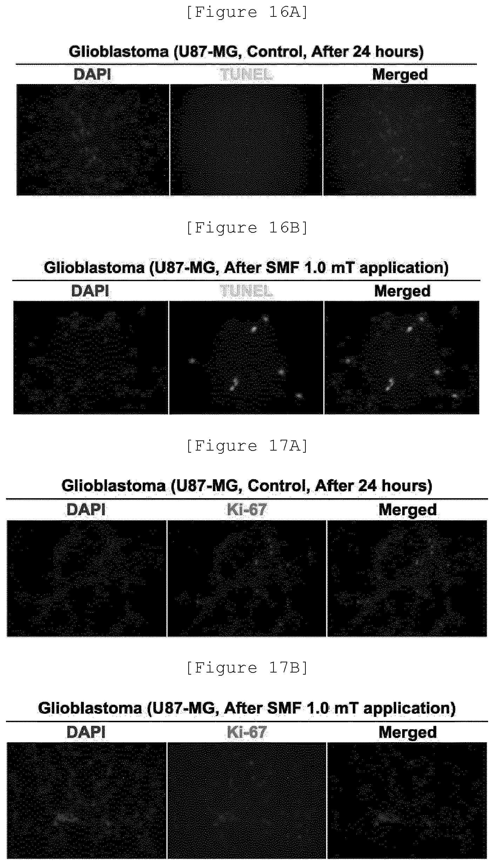

Next, when comparing results of TUNEL staining to check DNA fragmentation which is one of characteristics of cell dead, green fluorescence-stained portions appeared only in the cells to which the static magnetic field was applied. FIGS. 16A and 16B illustrate results of TUNEL staining, FIG. 16A illustrates a group (a control group) obtained by performing TUNEL staining after culturing was performed in an incubator for 24 hours without applying a static magnetic field, and FIGS. 16B illustrates a group (an experimental group) obtained by performing TUNEL staining after the static magnetic field was applied for 24 hours. It could be confirmed that a cell dead process was performed as a result of the TUNEL staining.

Finally, when comparing results of cell immunochemical staining to check Ki-67 which is a cell proliferation marker, a degree of expression of Ki-67 was low in the cells to which the static magnetic field was applied. FIGS. 17A and 17B illustrate results of cell immunochemical staining, and FIG. 17A illustrates a group (a control group) obtained by performing cell immunochemical staining after culturing was performed for 24 hours in an incubator without applying a static magnetic field, and FIG. 17B illustrates a group (an experimental group) obtained by performing cell immunochemical staining after a static magnetic field was applied for 24 hours.

As described above, when the results obtained through the cell fluorescence staining method are combined, the glioblastoma cell line U-87MG may be affected by a static magnetic field.

## Change in Tumor Cells in Static Magnetic Field: Protein Immunity Analysis

After a static magnetic field is applied for 24 hours, U87-MG cells were collected in the form of pellets and protein was isolated. Total protein was extracted by using PRO-PREP™ (iNiRON Biotechnology Inc., Seongnam-si, Gyeonggi-do, Korea). The amount of protein used was 10 μg, and sodium dodecyl sulfate-poly acrylamide gel electrophoresis (SDS-PAGE) was performed by selecting an appropriate gel percentage according to a size of the protein to be observed. Among genes related to cell dead, cleaved caspase-3, cleaved caspase-9 and cleaved PARP, which are cleaved forms, used 40 μg of protein, and phospho-Akt and phospho-PI3K, which are phosphorylated forms, and β-actin, which is a house-keeping gene, used 20 μg of protein.

Polyvinylidene fluoride (PVDF) (Whatman, Little Chalfont, Buckinghamshire, United Kingdom) was immersed in a methanol solution and activated, and then, a transfer process was performed for 1 hour and 20 minutes. Then, blocking was performed at room temperature for 1 hour with 1x tris-buffered saline with Tween 20 (TBS-T) including bovine serum albumin (BSA) (GenDEPOT, Katy, TX, United States) of 3% to minimize non-specific binding of antibody and protein, and the primary antibody was reacted at 4°C for 16 hours. The type of used primary antibody and a dilution ratio thereof were cleaved caspase-3 (1:500), caspase-3 (1:1000), cleaved caspase-9 (1:200), PARP (1:1000) (abcam, Cambridge, United Kingdom), caspase-9 (1:1000), cleaved PARP (1:500), phospho-Akt (1:500), Akt (1:1000), phospho-PI3K (1:500), PI3K (1 :1000) (Cell Signaling Technology, Inc., Danvers, MA, United States), and β-actin (1:500). Washing was done three times for 15 minutes with 1xTBS-T, and goat anti-rabbit IgG (H + L)-horseradish peroxidase (HRP) conjugate (Bio-Rad Laboratories, Inc., Hercules, CA, United States) was diluted at a ratio of 1:1000 and reacted at room temperature for 1 to 2 hours. Cleaved caspase-3, cleaved caspase-9, and cleaved PARP were diluted at a ratio of 1:500. After washing was done three times for 15 minutes with 1xTBS-T, enhanced chemiluminescence (ECL) (Bio-Rad Laboratories, Inc., Hercules, CA, United States) was sprayed on a membrane to detect a protein signal.

As a result, first, expression of protein related to cell apoptosis was confirmed.

In order to check that the glioblastoma cell line U87-MG actually undergoes cell dead and inhibits cell proliferation by a static magnetic field, a change in an expression level of cleaved caspase-3, cleaved caspase-9, and cleaved PARP were investigated through protein immunity analysis. When the cell dead is made, caspase-3 and caspase-9 genes and PARP genes are cleaved, and accordingly, caspase-3 and caspase-9 genes and PARP genes are used as important hallmarks to check cell dead. As confirmed from FIG. 17, when a static magnetic field was applied, the expression level of cleaved caspase-3, cleaved caspase-9, and cleaved PARP were increased, and it could be confirmed that cell dead occurred.

In addition, protein expression related to signal transduction was confirmed.

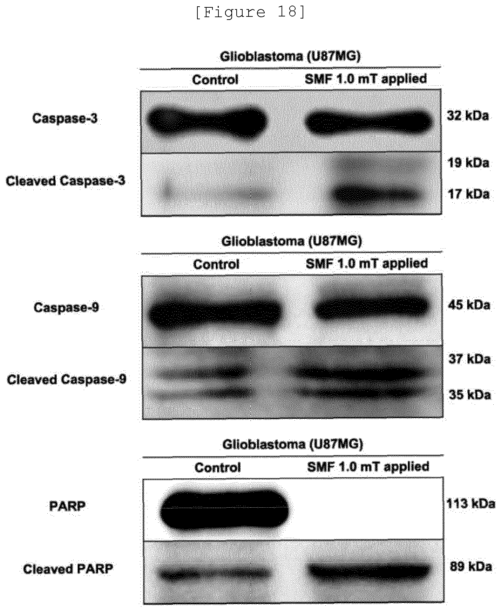

A signal transmission path of phosphatidylinositol-3-kinase (PI3K)/AKT (Protein Kinase B) is related to proliferation, growth, differentiation, migration, and cycle of cells and play an important role, and is also used as a targeted therapy targeting a PI3K/Akt path. Therefore, it was confirmed whether the signal transmission path is affected by a static magnetic field and a change in an expression level of PI3K phosphorylation and Akt phosphorylation through protein immunity analysis. As confirmed from FIG. 18, when a static magnetic field was applied, both PI3K phosphorylation and Akt phosphorylation were reduced, and through this, it was confirmed that cell proliferation is inhibited, and cell migration is also inhibited.

## Change in Tumor Cell in Static Magnetic Field: Western Blot Test Result

As in the present embodiment, dead of tumor cells according to application of a static magnetic field was also confirmed from a Western Blot test result.

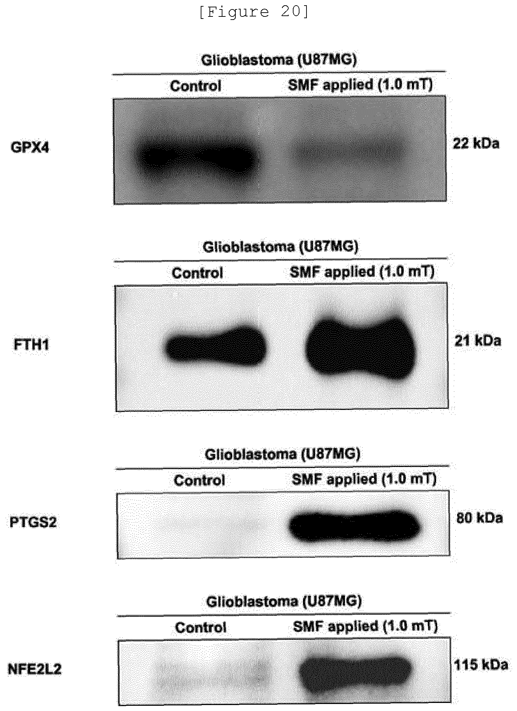

FIG. 20 illustrates a result of the Western blot experiment in which Ferroptosis, which is another mechanism of cell dead other than apoptosis, was significantly increased in a group of U87-MG tumor cells to which a static magnetic field of 1.0 mT according to the present embodiment was applied compared to a group of U87-MG tumor cells to which the static magnetic field was not applied.

It may be seen from the same drawing that, glutathione peroxidase 4 (GPX4), FTN1 (ferritin), prostaglandin-endoperoxide synthase 2 (PTGS2), nuclear factor erythroid2-related factor 2 (NFE2L2) observed from cells with remarkably increased ferroptosis was significantly increased in a group of U87-MG tumor cells to which a static magnetic field of 1.0 mT according to the present embodiment was applied compared to a group to which the static magnetic field was not applied.

**Change in Tumor Cells According to Change in Magnitude of Static Magnetic Field**

The embodiment described above relates to a change in cancer cells according to application of a static magnetic field of 1.0 mT. The present embodiment relates to a change in tumor cells according to application of a static magnetic field of 0.6 mT based on the same experimental condition, method, and so on.

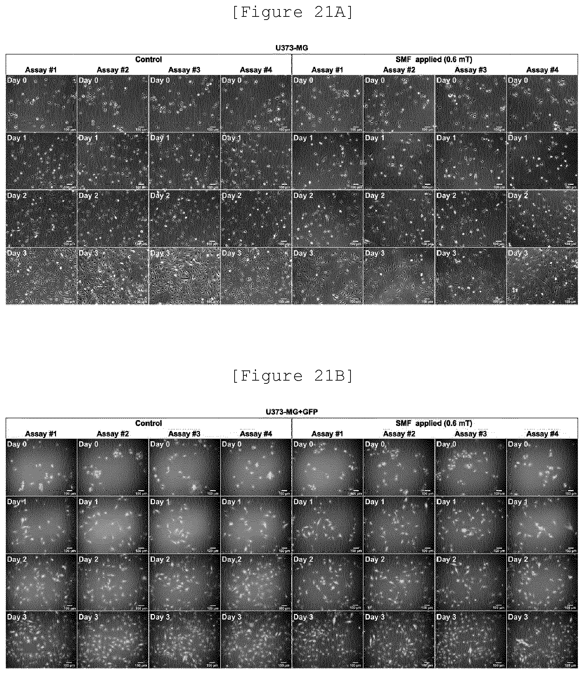

FIGS. 21A and 21B are photographs illustrating experimental results of tumor cell shapes when a static magnetic field of 0.6 mT according to the present embodiment was applied, the photographs being respectively taken by using an optical microscope and using a fluorescence microscope.

As may be seen from FIGS. 21A and 21B, the number of tumor cells in the left three control groups (control #1, control #2, and control #3) to which a static magnetic field was not applied is reduced compared to the number of tumor cells on day 0, day 1, day 2, and day 3, and the number of tumor cells in the right three SMF groups (SMF #1, SMF #2, and SMF #3) to which a static magnetic field of 0.6 mT was applied is reduced compared to the number of tumor cells on day 0, day 1, day 2, and day 3 at the same time.

This effect may be clearly seen when compared with an experimental result obtained by performing the same experiment on normal cells other than tumor cells.

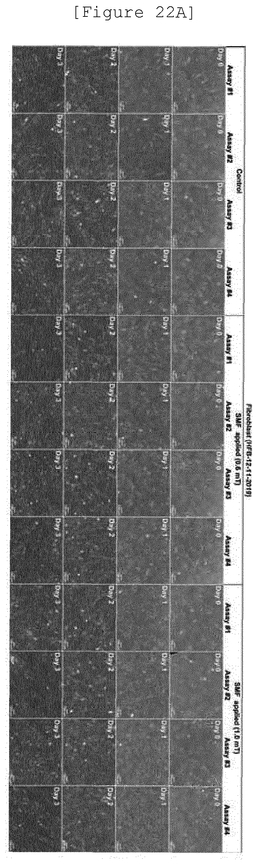

FIG. 22A illustrates photographs of appearances of normal cells (fibroblasts) other than tumor cells in a control group to which a static magnetic field was not applied, an SMF group to which an SMF magnetic field of 0.6 mT was applied, and an SMF group to which an SMF magnetic field of 1 mT was applied, and FIG. 21B is a graph illustrating a change in the number of different cells over time in each group.

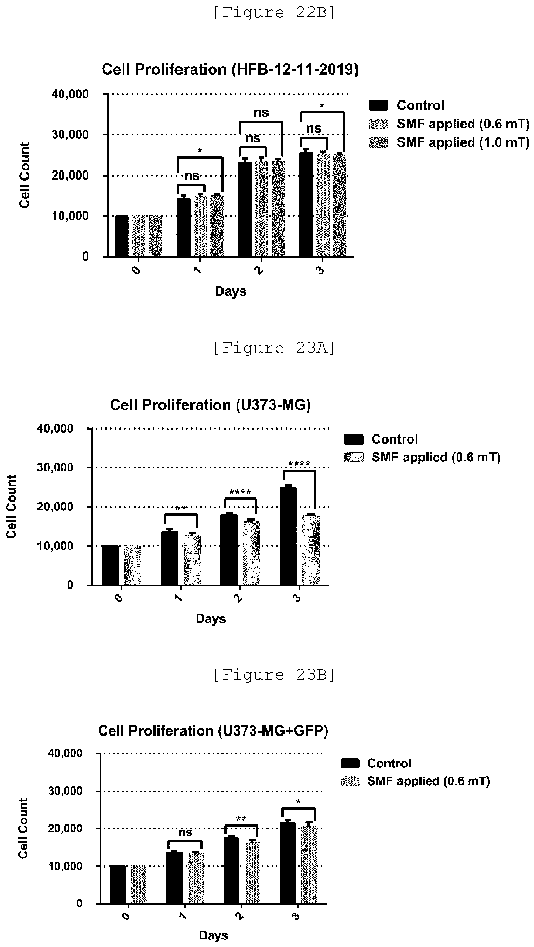

As may be seen from FIGS. 22A and 22B, it may be confirmed that no abnormality was observed in cells even when static magnetic fields of 0.6 mT and 1.0 mT were applied to normal cells (fibroblasts) other than tumor cells.

That is, it may be seen from FIGS. 21A and 21B and FIGS. 22A and 22B that a static magnetic field, which have no effect on normal cells, acts on tumor cells to cause cell dead to occur even under a very small static magnetic field of 0.6 mT in the present embodiment, and accordingly, the number of tumor cells is reduced.

FIGS. 23A and 23B illustrate results of measuring the number of cells in an experimental group and a control group through trypan blue staining.

$1 \times 104$ cells were planted in a culture dish of 35 mm at the beginning of an experiment for each of an experimental group to which a static magnetic field was applied and a control group to which the static magnetic field was not applied. The static magnetic field was applied after about 12 hours from when cells were planted, and was continuously applied until 72 hours after the experiment was completed.

After 24 hours from when the experiment started, the number of U373-MG cells in the control group and the number of U373-MG cells in the experimental group were respectively $1.37 \times 104$ and $1.25 \times 104$, and after 48 hours from when the experiment started, the number of U373-MG cells in the control group and the number of U373-MG cells in the experimental group were respectively $1.78 \times 104$ and $1.61 \times 104$, and a difference between the two groups began to appear. Finally, after 72 hours, the number of cells in the control group and the number of cells in the experimental group were respectively $2.48 \times 104$ and $1.76 \times 104$, and the difference therebetween was further increased.

After 24 hours from when the experiment started, the number of U373-MG+GFP cells in the control group and the number of U373-MG+GFP cells in the experimental group were respectively $1.35 \times 104$ and $1.33 \times 104$, and after 48 hours from when the experiment started, the number of U373-MG+GFP cells in the control group and the number of U373-MG+GFP cells in the experimental group were respectively $1.73 \times 104$ and $1.63 \times 104$, and a difference between the two groups began to appear. Finally, after 72 hours, the number of cells in the control group and the number of cells in the experimental group were respectively $2.15 \times 104$ and $2.04 \times 104$, and the difference therebetween was further increased.

The number of U373-MG cells in the experimental group increased at an average rate of $0.25 \times 104$ cells per day and the number of U373-MG cells in the control group increased at an average rate of $0.49 \times 104$ cells per day, and the number of U373-MG+GFP cells in the experimental group increased at an average rate of $0.35 \times 104$ cells per day and the number of U373-MG+GFP cells in the control group increased at an average rate of $0.38 \times 104$ cells per day. Therefore, it may be seen that the static magnetic field of 0.6 mT acts to inhibit the cell proliferation of U373-MG cells and U373-MG+GFP cells, which are glioblastoma cell lines. In addition, it may be seen from experimental results of U373-MG cells and U373-MG+GFP cells that the effect of the static magnetic field of 0.6 mT changes depending on cells.

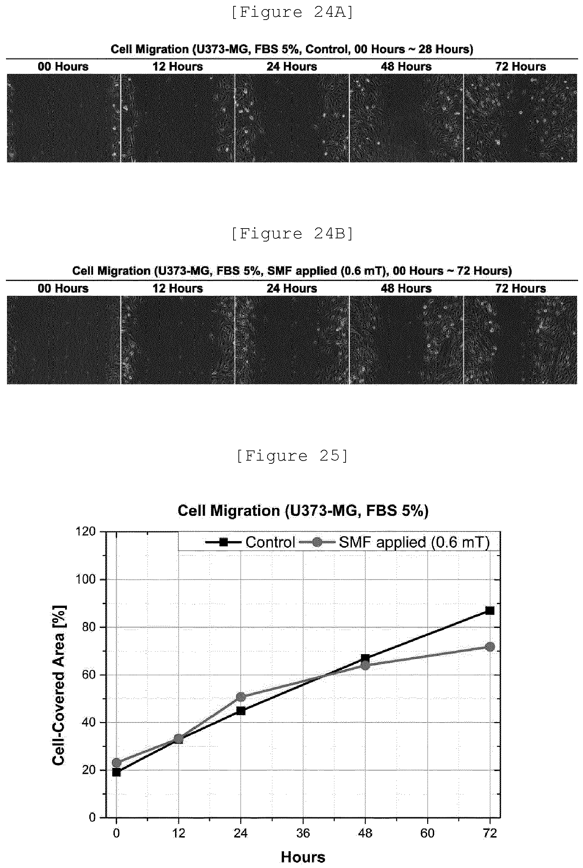

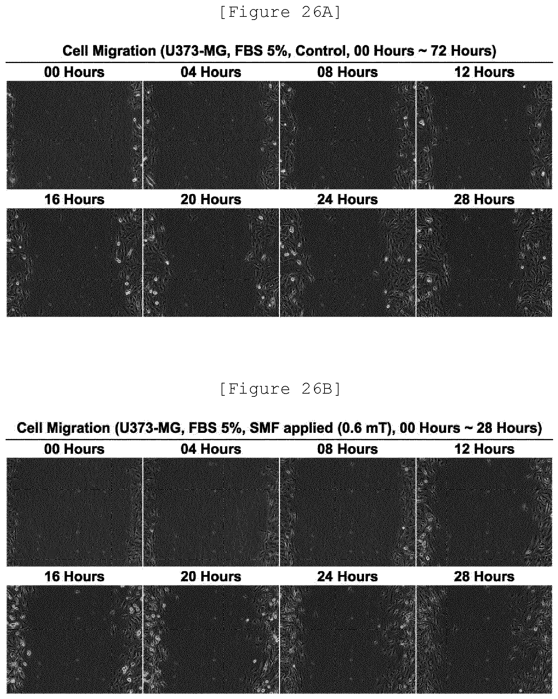

FIGS. 24A and 24B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 5% was added. FIG. 25 is a graph in which cell-covered areas of micrographs of FIGS. 24A and 24B are obtained by using Image J. FIGS. 26A and 26B are photographs obtained by subdividing at 4-hour intervals the micrographs of FIGS. 24A and 24B from 0 to 28 hours.

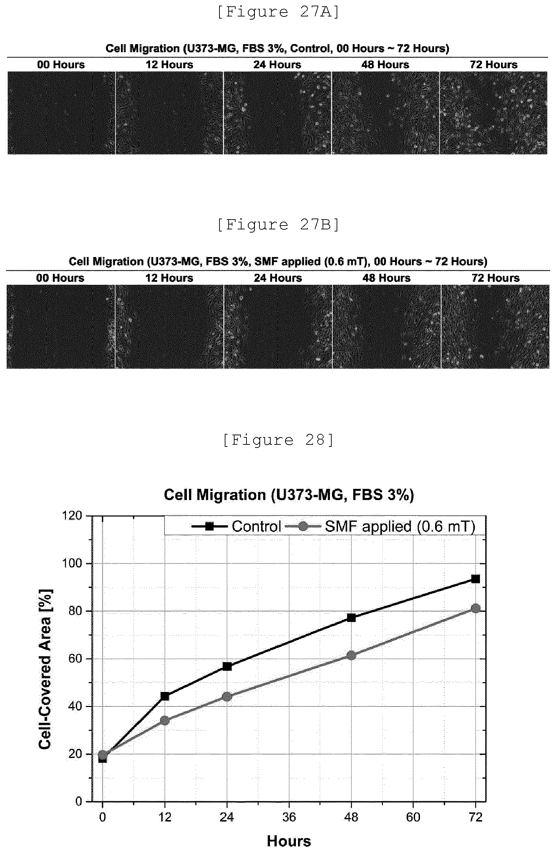

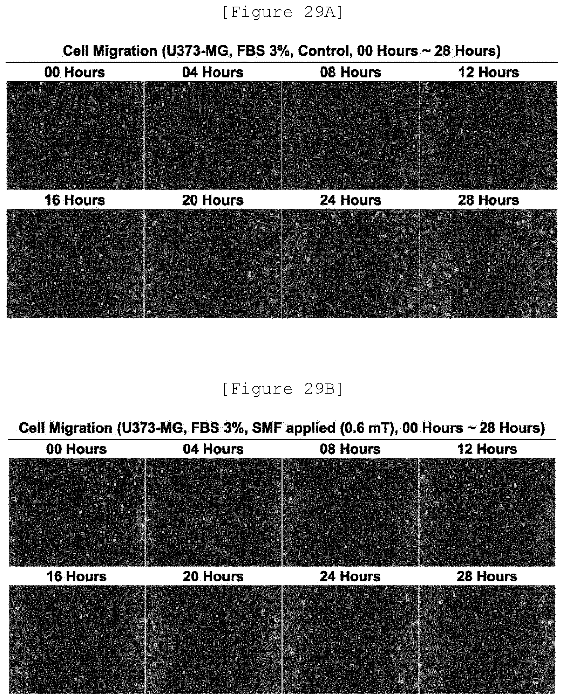

FIGS. 27A and 27B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 3% was added. FIG. 28 is a graph in which cell-covered areas of micrographs of FIGS. 27A and 27B are obtained by using Image J. FIGS. 29A and 29B are photographs obtained by subdividing at 4-hour intervals the micrographs of FIGS. 27A and 27B from 0 to 28 hours.

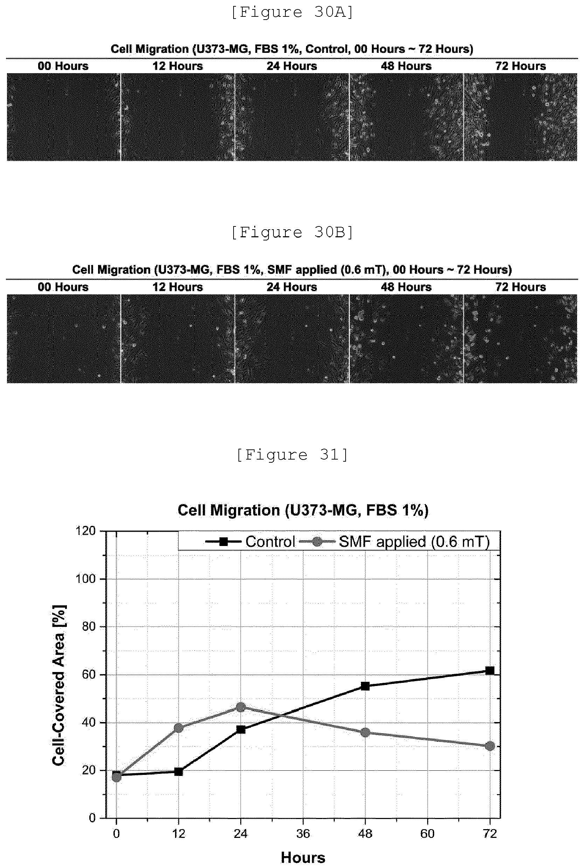

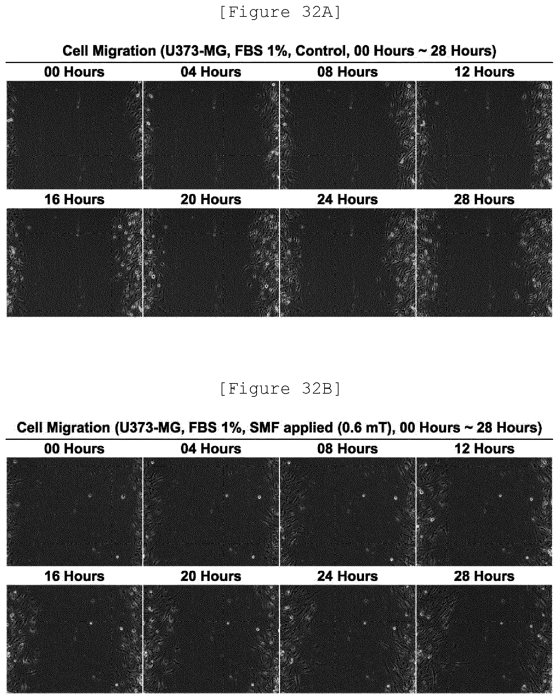

FIGS. 30A and 30B illustrate photographs taken under a microscope of an experimental group (a static magnetic field of 0.6 mT is applied) and a control group (a static magnetic field is not applied) when 0 hour, 24 hours, 48 hours, and 72 hours elapsed after a cell migration experiment of glioblastoma U373-MG was performed with a culture fluid to which FBS of 1% was added. FIG. 31 is a graph in which cell-covered areas of micrographs of FIGS. 30A and 30B are obtained by using Image J. FIGS. 32A and 32B are photographs obtained by subdividing at 4-hour intervals the micrographs of FIGS. 30A and 30B from 0 to 28 hours.

### Effect of Static Magnetic Field on Various Cancer Cells

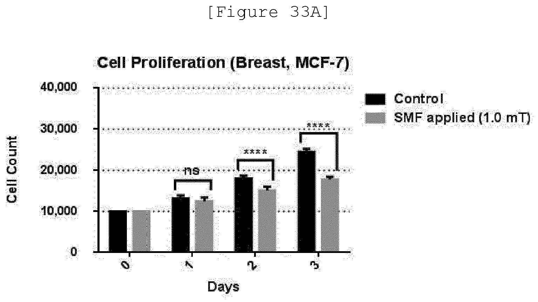

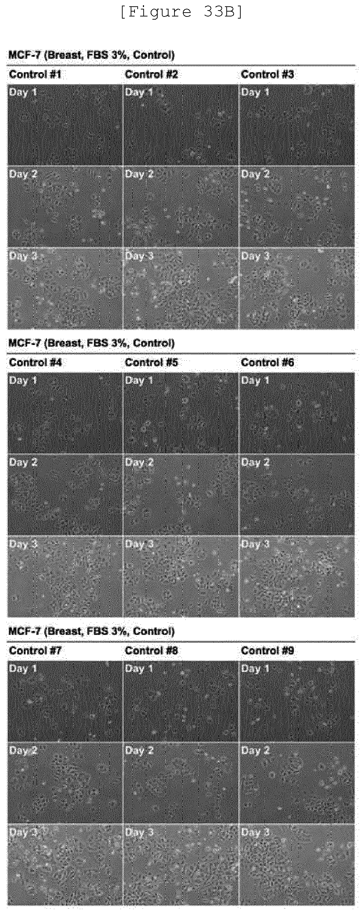

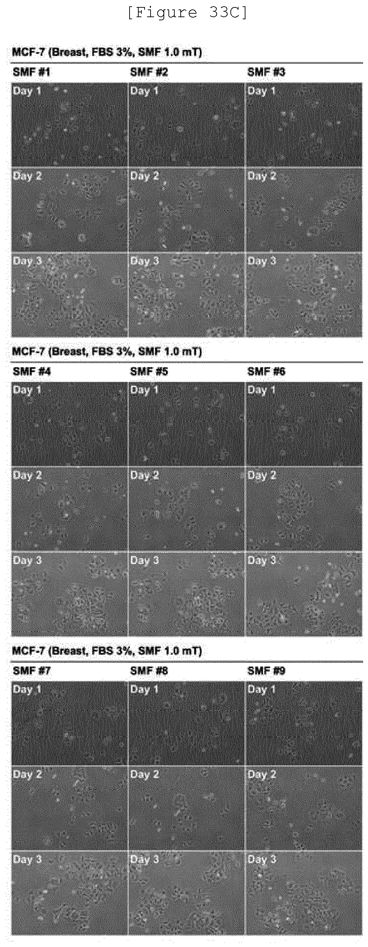

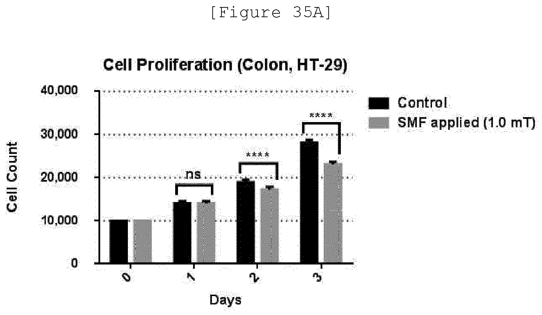

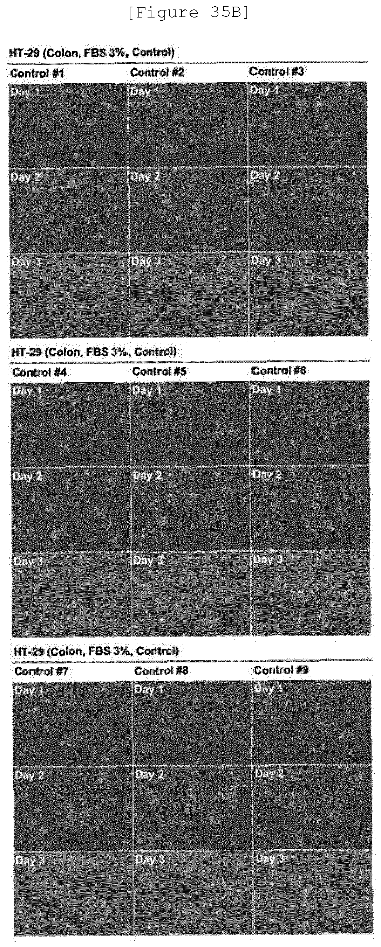

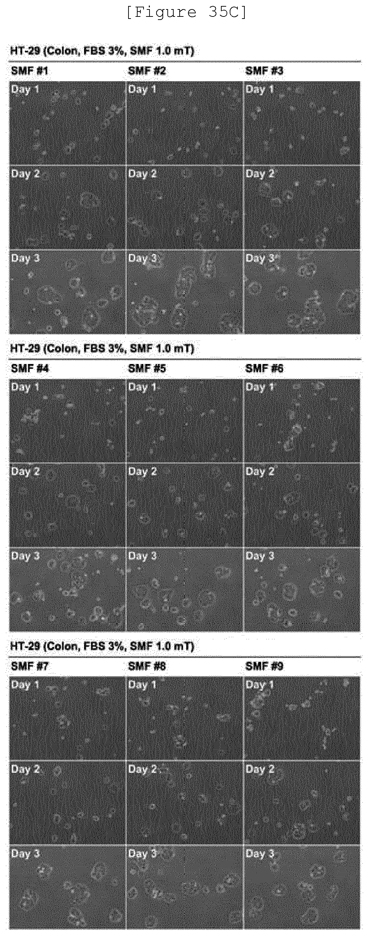

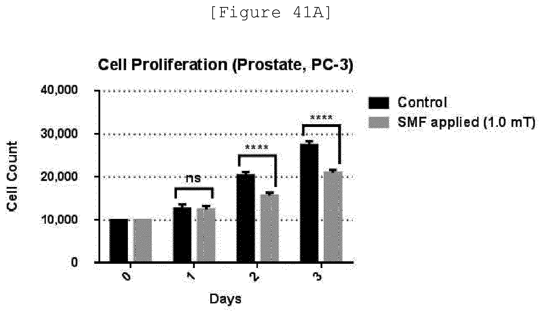

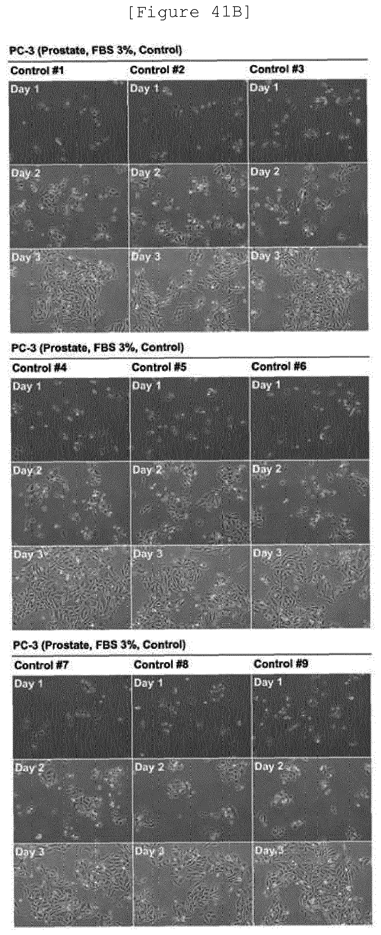

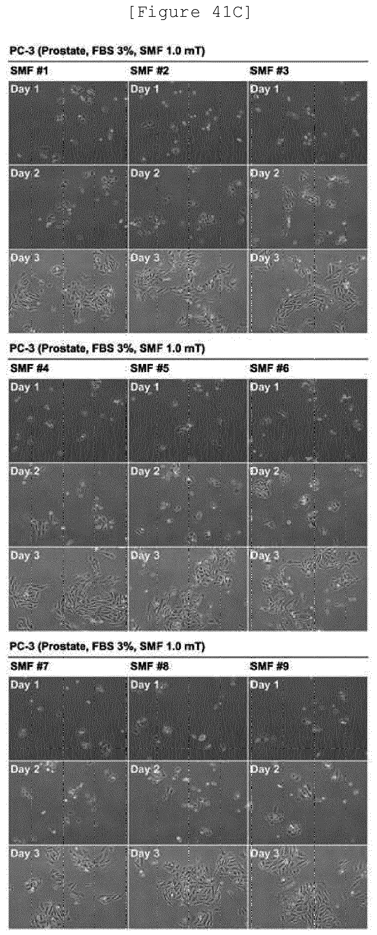

FIGS. 33A to 44C illustrate that a static magnetic field according to an embodiment of the present disclosure is effective on not only the specific tumor cells described above but also 12 types of tumor cells below.

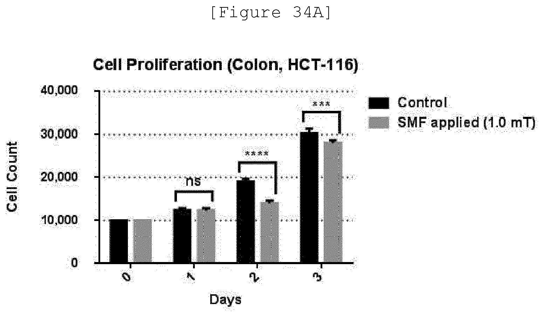

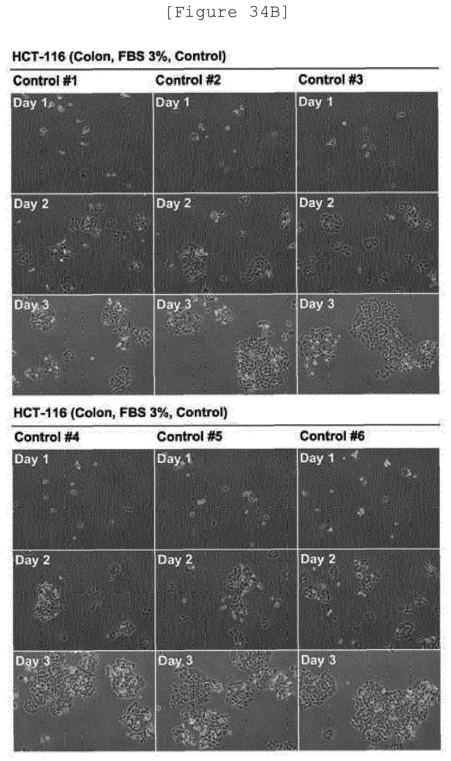

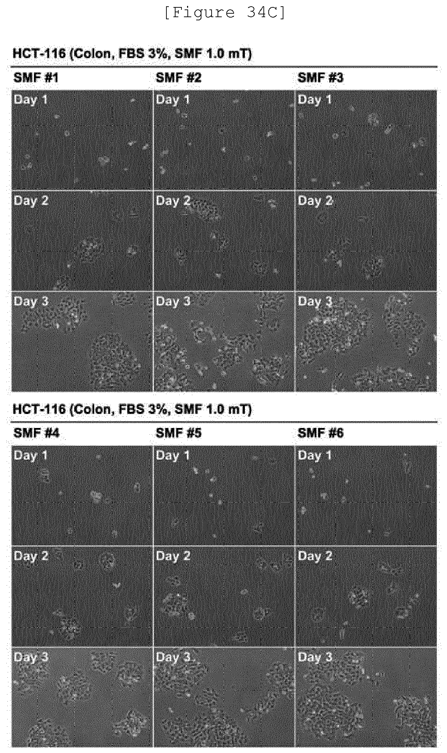

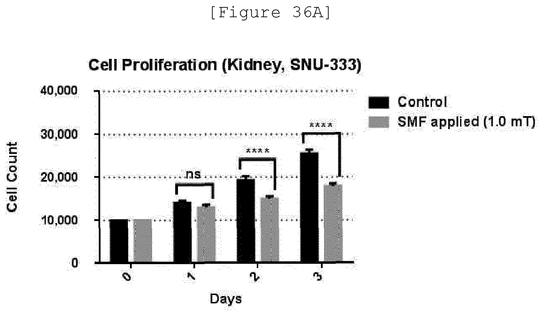

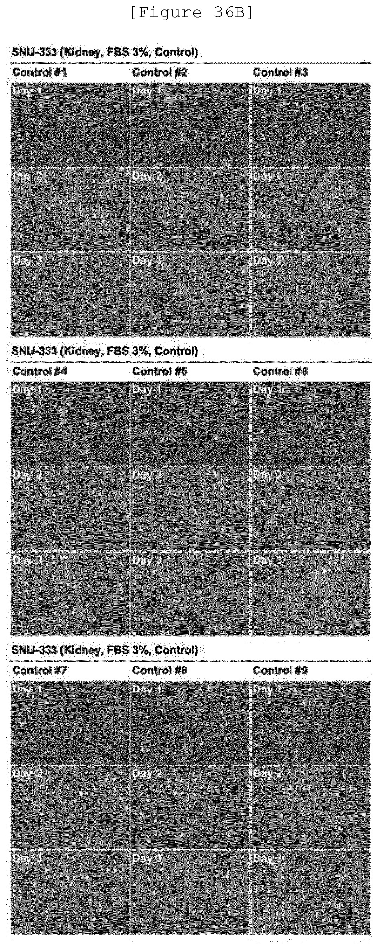

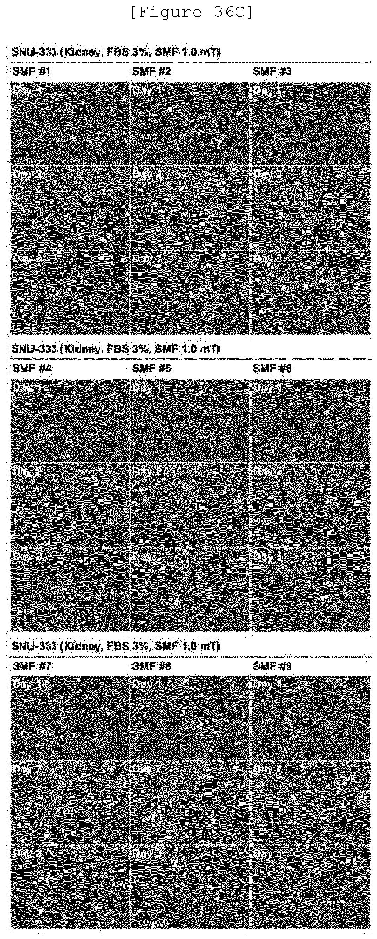

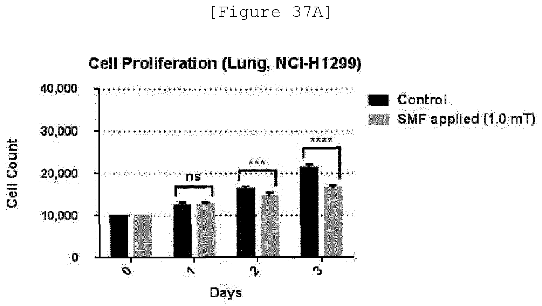

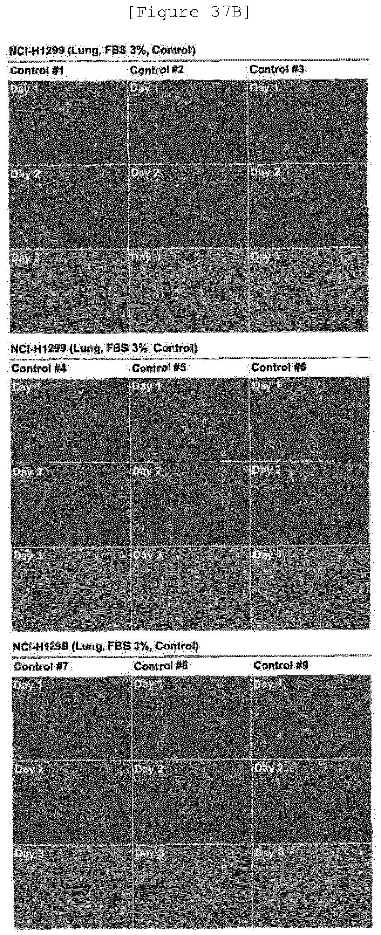

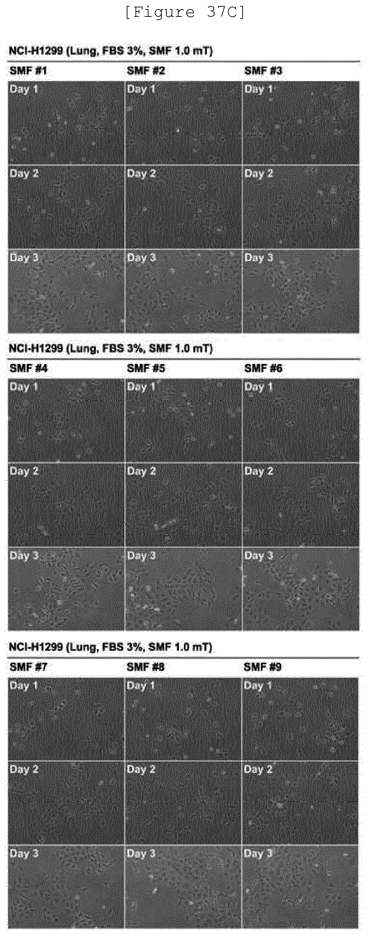

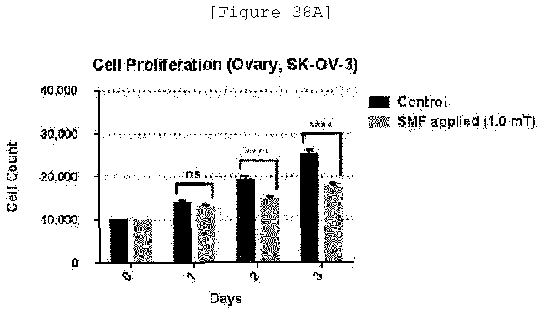

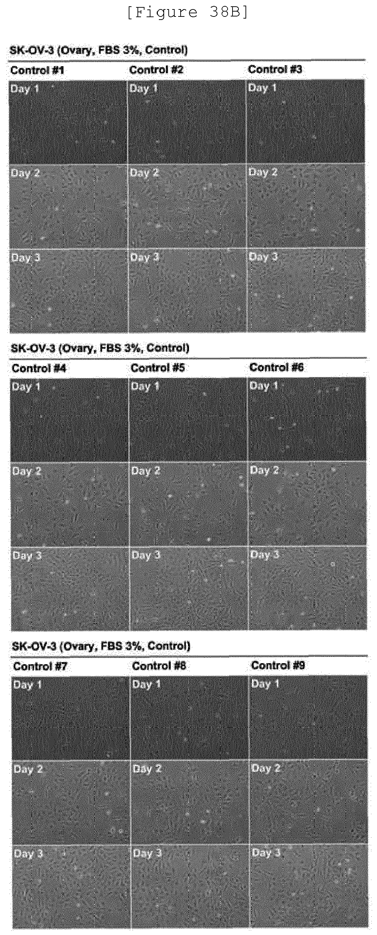

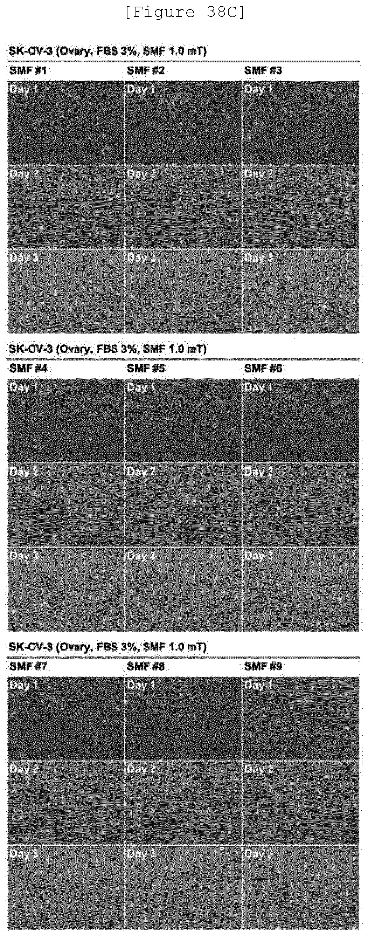

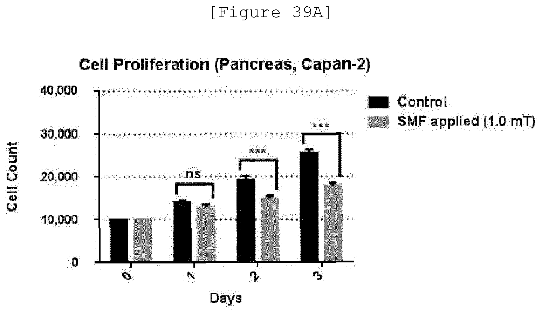

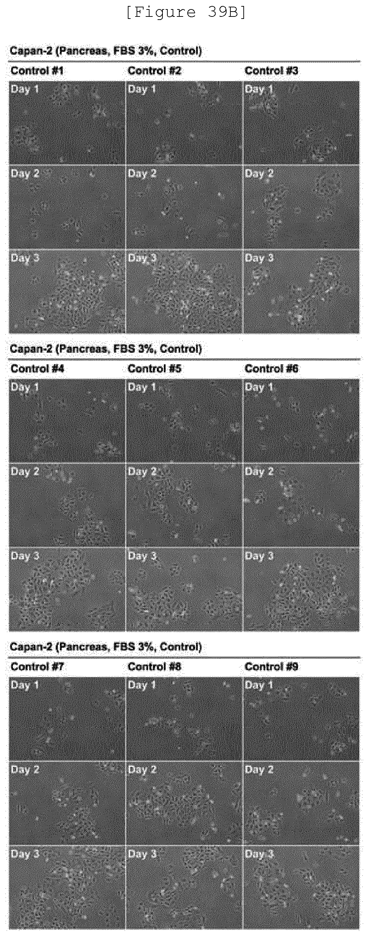

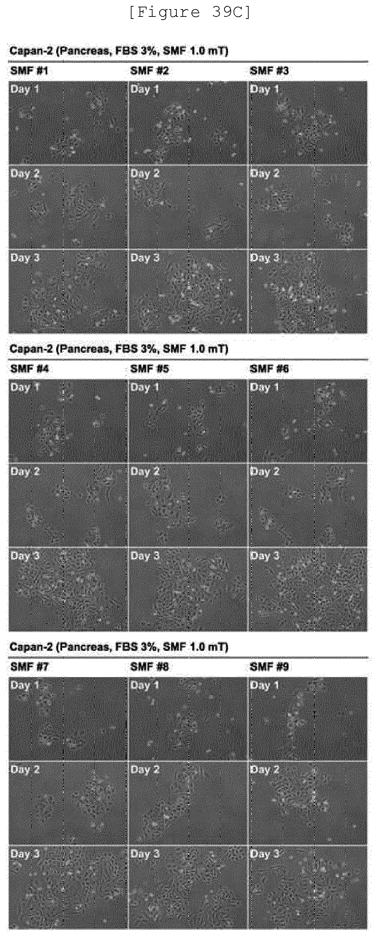

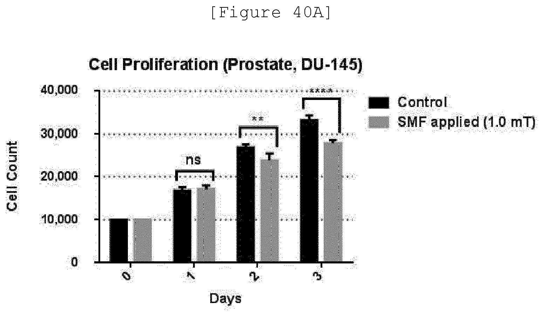

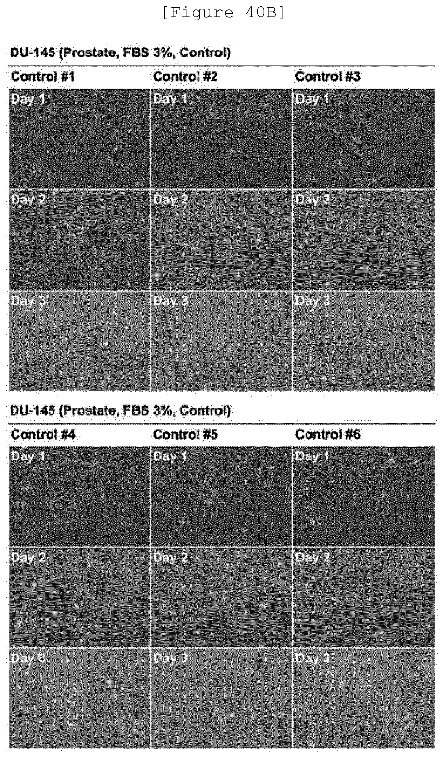

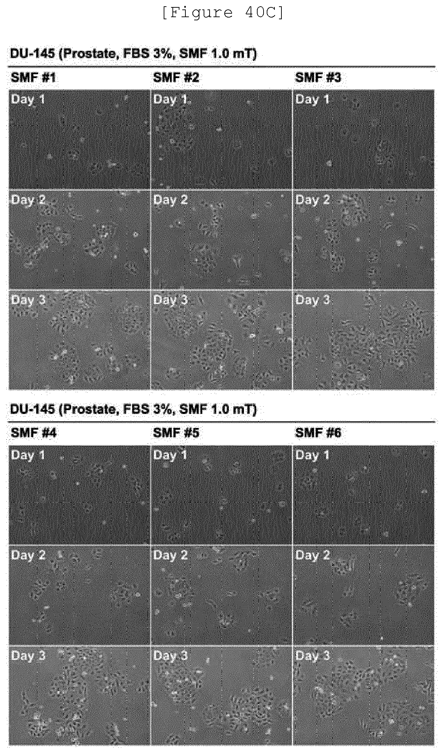

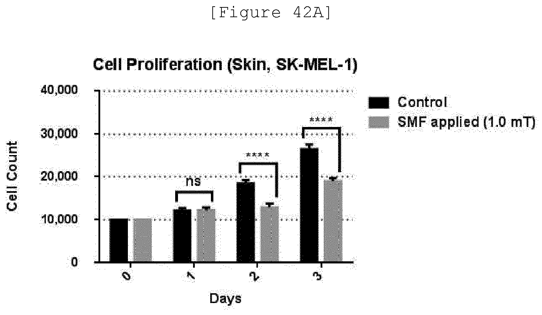

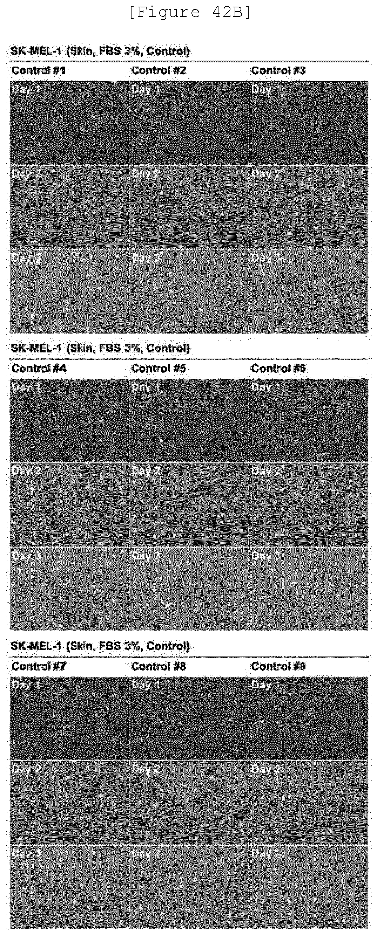

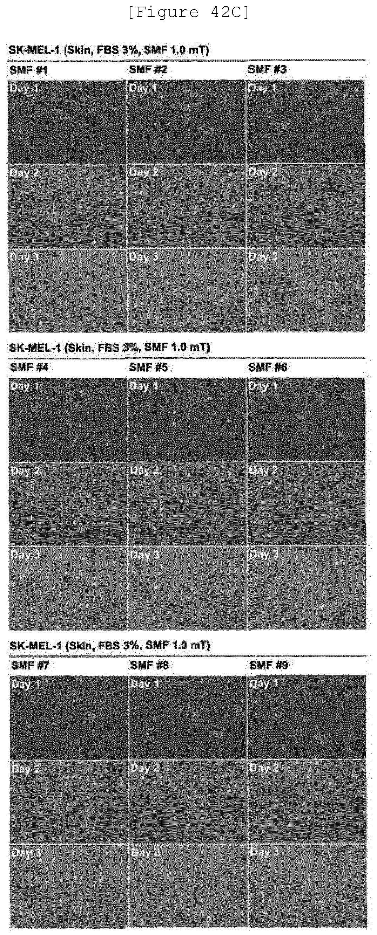

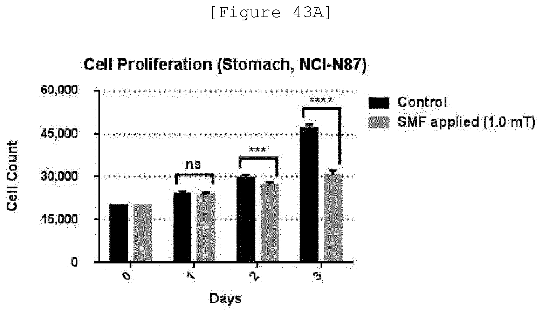

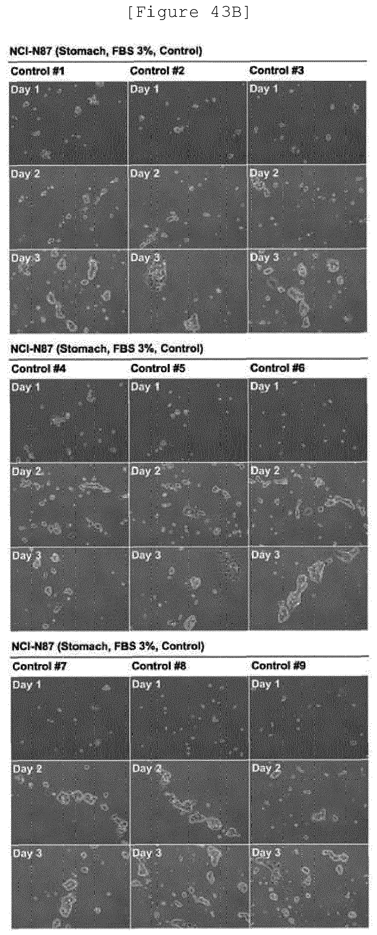

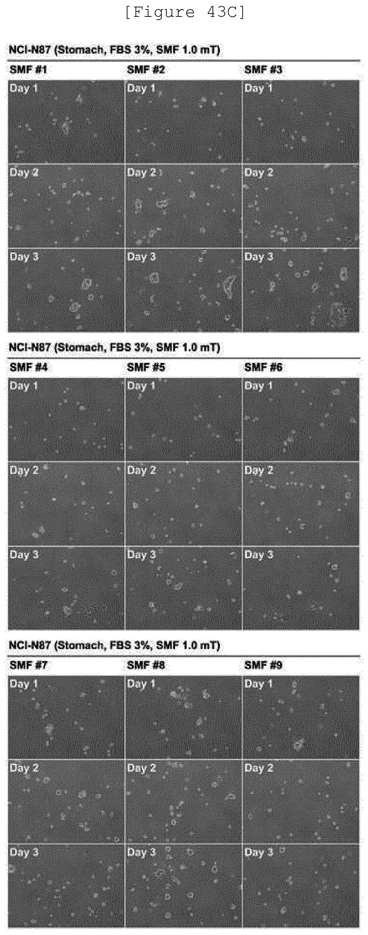

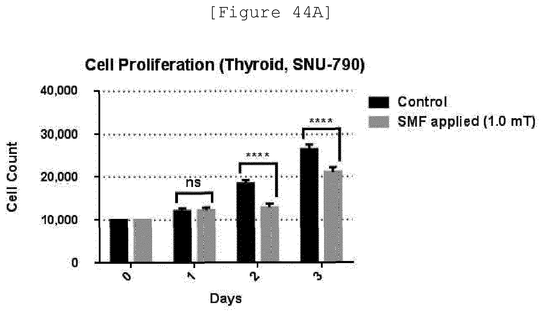

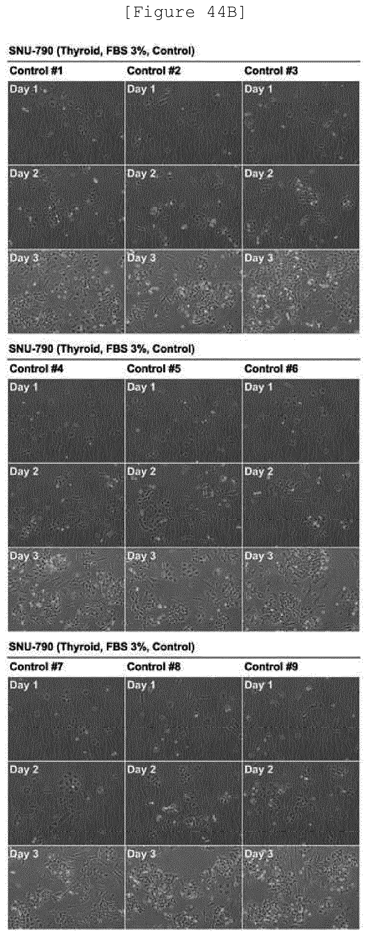

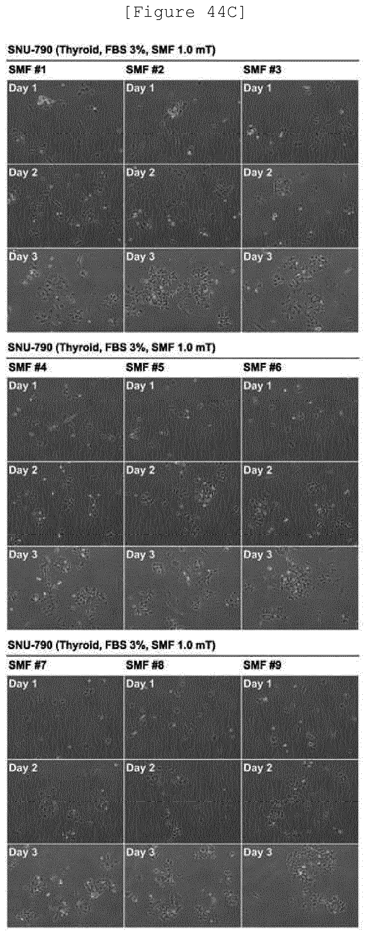

1) Breast cancer (MCF-7 cell line): FIGS. 33A to 33C
2) Colon cancer (HCT-116 cell line and HT-29 cell line): FIGS. 34A to 35C
3) Renal cancer (SNU-333 cell line): FIGS. 36A to 36C
4) Lung cancer (NCI-H1299 cell line): FIGS. 37A to 37C
5) Ovarian cancer (SK-OV-3 cell line): FIGS. 38A to 38C
6) Pancreatic cancer (Capan-2 cell line): FIGS. 39A to 39C
7) Prostate cancer (DU-145 cell line, PC-3 cell line): FIGS. 40A to 41C
8) Skin cancer (SK-MEL-1 cell line): FIGS. 42A to 42C
9) Gastric cancer (NCI-N87 cell line): FIGS. 43A to 42C
10) Thyroid cancer (SNU-790 cell line): FIGS. 44A to 44C

FIGS. 33A to 44C illustrate results (A figures of FIGS. 33A to 44C) of measuring the number of cells in the experimental group (a static magnetic field of 1.0 mT is applied) and the control group (a static magnetic field is not applied) through trypan blue staining for each of the 12 types of cancer cell lines and results of observing shapes of the cells in the control group (B figures of FIGS. 33A to 44C) and the experimental group (C figures of FIGS. 33A to 44C), and correspond to photographs showing cells reduced by applying a static magnetic field of 1 mT for 3 days under a culture condition of FBS of 3% compared to a control group and correspond to statistically processed data by counting the number of cells.

It may be seen from FIGS. 33A to 44C that the number of cells and cell migration are significantly reduced as a static magnetic field of 1 mT is applied to the 12 types of cancers according to the present embodiment.

Various Embodiments of Static Magnetic Field Generator

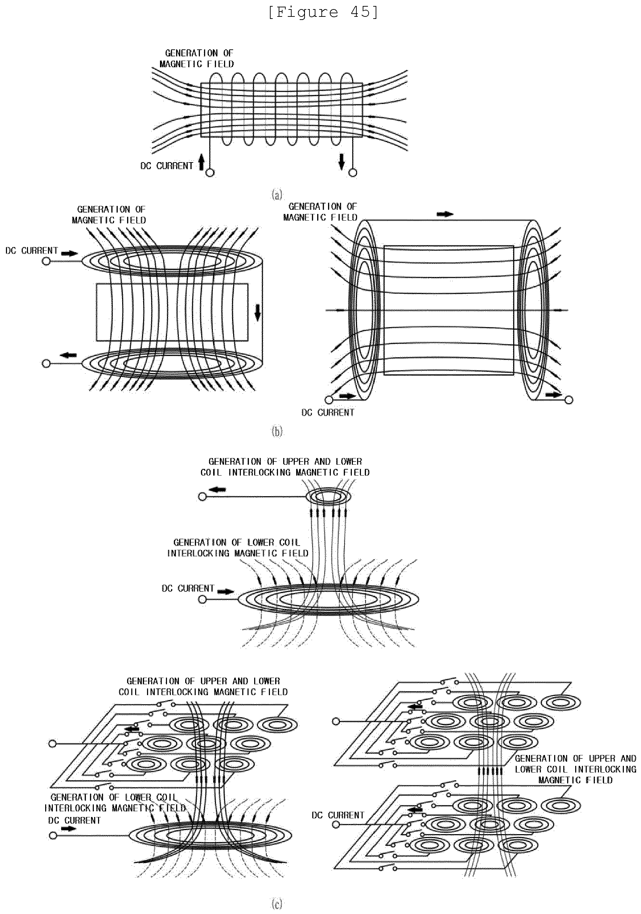

FIGS. 45 to 47 illustrate various embodiments of a static magnetic field generator applicable to the present disclosure.

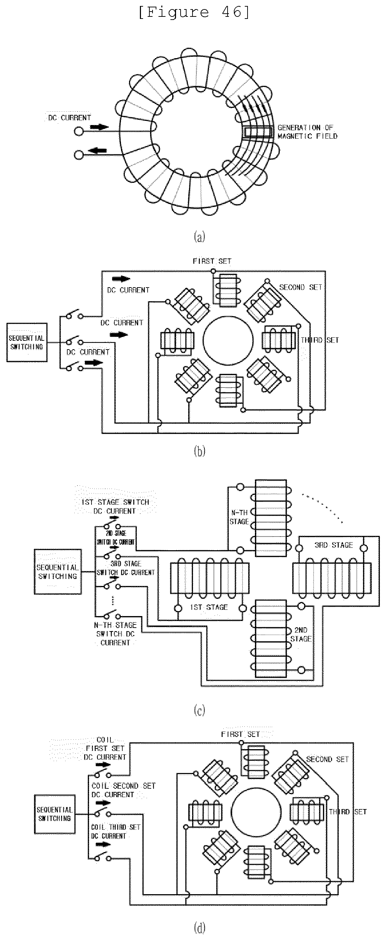

Specifically, FIGS. 45 and 46 illustrate embodiments of a static magnetic field generators using a direct current (DC), according to various types of structures below.

1) Type 1: simple solenoid structure (see (a) of FIG. 45)
2) Type 2/3: up/down/left/right Helmholtz structure (see (b) of FIG. 45)
3) Type 4: upper and lower coil selection Helmholtz magnetic field focusing structure (see (c) of FIG. 45)
4) Type 5: core coil structure (see (a) of FIG. 46)
5) Type 6: 3-stage set coil multi-phase structure (see (b) of FIG. 46)
6) Type 7: N-stage multi-coil multi-phase structure (see (c) of FIG. 46)
7) Type 8: core coil multi-frequency application rotation structure (see (d) of FIG. 46)

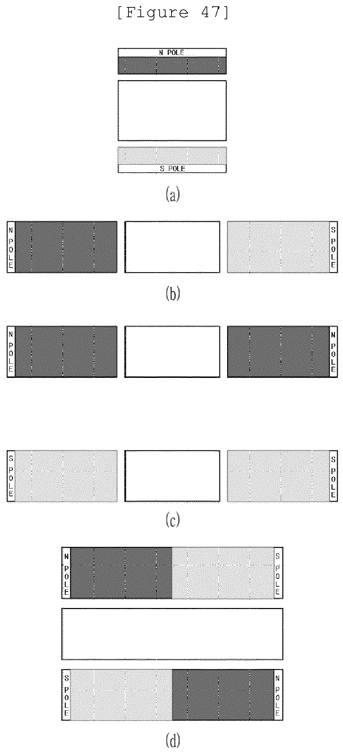

In addition, a static magnetic field may be generated by using a permanent magnet instead of a DC current, and various embodiments related thereto are illustrated in FIG. 47.

In addition, an operation and a magnetic field generation principle of each of the static magnetic field generator structures illustrated in FIGS. 45 to 47 will be obvious to those skilled in the art, and thus, detailed descriptions thereof are omitted.

A summary of the embodiments described above is as follows.

According to one embodiment, it may be seen that there is a change when a static magnetic field of a certain size is applied to various types of cancer including glioblastoma cell lines. In an example for generating a static magnetic field of a uniform size, a structure of the Helmholtz coil is used, and cell proliferation may be checked by directly measuring the number of cells changed by a static magnetic field through trypan blue staining, and a change in shapes of cells may also be checked through a microscope. In addition, cell live and dead may be checked through cell fluorescence staining, the cell live and dead may be checked through calcein-AM staining, and cell dead may be checked through TUNEL staining. Finally, expression of Ki-67 may also be checked through cell immunochemical staining.

The Helmholtz coil used in the embodiment is designed with parameters, such as a diameter of a coil required for design, the number of turns of a coil, and a magnitude of a current to flow through the coil, and two circular coils are placed vertically in an upper portion and a lower portion in the z-axis direction.

As a result, for example, when a static magnetic field of 0.6 mT or 1.0 mT is applied to a glioblastoma cell line, the number of cells is hardly changed, and a shape of the cell is slightly more elongated compared to a shape of the cell when the magnetic field is not applied. In a cell migration experiment, concentrations of FBS to be added are set to 5%, 3%, and 1% in order to reduce various effects caused by the FBS as much as possible, cell migration begins to slow down after about 12 hours under conditions in a culture solution including concentration of FBS of 3%, and later, the speed of filling empty parts is also reduced gradually. Cell dead is observed while cell migration is inhibited, and accordingly, calcein-AM staining is performed to check cell live and dead, TUNEL staining is performed to check cell dead, and Ki-67 staining is performed to check a cell proliferation marker. As a result, staining results with characteristics of cell dead are obtained only when a static magnetic field is applied. In addition, a protein expression experiment was performed, and protein expression of cleaved caspase-3, cleaved caspase-9, and cleaved PARP, which are cleaved forms of genes related to cell dead, was increased in cells to which a static magnetic field was applied. In addition, protein expression of PI3K phosphorylation and Akt phosphorylation, which are phosphorylated forms, among genes related to signal transmission, was reduced in the cells to which a static magnetic field was applied.

Therefore, when summarizing all these molecular biological results, it may be seen that a static magnetic field inhibits cell proliferation and cell migration of glioblastoma cell lines and induces cell dead.

In particular, the magnitude of a static magnetic field according to an embodiment of the present disclosure ranges from 0.1 mT to 4 T, where 0.1 mT is a minimum magnitude required for removing and inhibiting cancer cells, and when exceeding 4 T, the static magnetic field may cause harm to the human body. For example, a magnetic flux density of 0.1 mT in the present embodiment corresponds to a very weak static magnetic field that is "30,000 times" lower than the magnetic flux density of 4 T used in a magnetic resonance imaging device. Although a static magnetic field of 1 mT which is another exemplary size is harmless to the human body and has very small magnitude of about "1/4,000" compared to the magnitude of a static magnetic field of 4 T which is an upper limit of the discovered magnitude, tumors are killed and tumor invasion is significantly reduced, and even under continuous treatment, safety to the human body and cancer treatment efficacy may be ensured at the same time.

In addition, when a static magnetic field of 1.0 mT is applied to tumor cells illustrated in the embodiment of the present disclosure, rather than when a static magnetic field of 0.6 mT is applied, more tumors are killed, and tumor invasion is more significantly reduced, and thus, the larger the magnitude of a static magnetic field, the greater the effect on the tumors.

Furthermore, even when a small static magnetic field of about 1.0 mT of the same size is applied to various types of cancer illustrated in the embodiments of the present disclosure, more tumors are killed in proportion to a duration of exposure, and tumor invasion is more significantly reduced, and thus, cancer treatment may be efficiently done by using a very low-level static magnetic field that is harmless to the human body even when applied for a long time.

Although the present disclosure is described in detail above, the scope of the present disclosure is not limited thereto, and it will be apparent to those skilled in the art that various modifications and variations may be made without departing from the technical idea of the present disclosure described in claims.

[Description of symbols]

110: first coil unit
111: first central axis
115: first coil housing
120: second coil unit

121: second central axis
125: second coil housing
130: treatment space

**Claims**

1. A static magnetic field generator for treatment of cancer, the static magnetic field generator comprising:

   a static magnetic field generating unit; and
   a space in which a static magnetic field is formed by the static magnetic field generating unit,
   wherein the formed static magnetic field does not substantially act on normal cells in the space and acts on cancer cells to be killed.

2. The static magnetic field generator of claim 1, wherein the formed static magnetic field has a magnitude range of 0.1 mT to 4 T.

3. A static magnetic field generator for treatment of cancer, the static magnetic field generator comprising:

   a static magnetic field generating unit; and
   a space in which a static magnetic field is formed by the static magnetic field generating unit,
   wherein a magnitude of the formed static magnetic field is configured to have a range of 0.1 mT to 4 T.

4. The static magnetic field generator of any one of claims 1 to 3, wherein the static magnetic field is configured to be generated by a direct current.

5. The static magnetic field generator of claim 4, wherein the static magnetic field generating unit has at least one of a solenoid structure, a vertical or horizontal Helmholtz structure, a core coil structure, a multi-stage set coil multi-phase structure, an upper or lower coil selection Helmholtz magnetic field focusing structure, a multi-stage multiple coil multi-phase structure, and a core coil multi-frequency application rotation structure.

6. The static magnetic field generator of claim 4, wherein

   the static magnetic field generating unit includes a plurality of coil units, and
   a space in which the static magnetic field is formed is placed in a space between the plurality of coil units.

7. The static magnetic field generator of claim 6, wherein the plurality of coil units comprises:

   a first coil unit in which a cylindrical coil is wound multiple times around a first central axis; and
   a second coil unit which is separated from the first coil by a preset distance and in which a cylindrical coil is wound multiple times around a second central axis coaxial with the first central axis.

8. The static magnetic field generator of claim 7, wherein

   currents of a same magnitude flows through the first coil unit and the second coil unit in the same direction, and
   a treatment region of a body is placed in a space between the first coil unit and the second coil unit.

9. The static magnetic field generator of claim 8, wherein the first coil unit and the second coil unit have coils of a same thickness which are wound around a same area in a same number of turns with same inner and outer diameters.

10. The static magnetic field generator of claim 7, further comprising:

    a first coil housing configured to surround and support the first coil unit; and
    a second coil housing configured to surround and support the second coil unit.

11. The static magnetic field generator of any one of claims 1 to 3, further comprising:
    a camera configured to image the space in which the static magnetic field is formed.

12. A static magnetic field generator for treatment of cancer, the static magnetic field generator comprising:

a plurality of coil units configured to generate a static magnetic field for a treatment region of a body, wherein a treatment region of the body is placed in a space between the plurality of coil units.

13. The static magnetic field generator of claim 12, wherein

a magnitude of the generated static magnetic field is configured to have a range of 0.1 mT to 4 T, and a space in which the static magnetic field is formed is placed in the space between the plurality of coil units.

14. The static magnetic field generator of claim 13, wherein the plurality of coil units comprises:

a first coil unit in which a cylindrical coil is wound multiple times around a first central axis; and a second coil unit which is separated from the first coil by a preset distance and in which a cylindrical coil is wound multiple times around a second central axis coaxial with the first central axis.

15. The static magnetic field generator of claim 14, wherein

currents of a same magnitude flows through the first coil unit and the second coil unit in the same direction, and a treatment region of a body is placed in a space between the first coil unit and the second coil unit.

16. The static magnetic field generator of claim 15, wherein the first coil unit and the second coil unit have coils of a same thickness which are wound around a same area in a same number of turns with same inner and outer diameters.

[Figure 1]

[Figure 2]

[Figure 3]

[Figure 4A]

Cell Proliferation (U87-MG)

[Figure 4B]

Cell Proliferation (U87-MG+GFP)

[Figure 5A]

[Figure 5B]

[Figure 6A]

**Cell Migration (U87-MG, FBS 5%, Control, 00 Hours ~ 72 Hours)**

| 00 Hours | 12 Hours | 24 Hours | 48 Hours | 72 Hours |

[Figure 6B]

**Cell Migration (U87-MG, FBS 5%, SMF applied (1.0 mT), 00 Hours ~ 72 Hours)**

| 00 Hours | 12 Hours | 24 Hours | 48 Hours | 72 Hours |

[Figure 7]

**Cell Migration (U87-MG, FBS 5%)**

[Figure 8A]

**Cell Migration (U87-MG, FBS 5%, Control, 00 Hours ~ 28 Hours)**

[Figure 8B]

**Cell Migration (U87-MG, FBS 5%, SMF applied (1.0 mT), 00 Hours ~ 28 Hours)**

[Figure 9A]

**Cell Migration (U87-MG, FBS 3%, Control, 00 Hours ~ 72 Hours)**

| 00 Hours | 12 Hours | 24 Hours | 48 Hours | 72 Hours |

[Figure 9B]

**Cell Migration (U87-MG, FBS 3%, SMF applied (1.0 mT), 00 Hours ~ 72 Hours)**

| 00 Hours | 12 Hours | 24 Hours | 48 Hours | 72 Hours |

[Figure 10]

**Cell Migration (U87-MG, FBS 3%)**

[Figure 11A]

**Cell Migration (U87-MG, FBS 3%, Control, 00 Hours ~ 28 Hours)**

| 00 Hours | 04 Hours | 08 Hours | 12 Hours |
|----------|----------|----------|----------|

| 16 Hours | 20 Hours | 24 Hours | 28 Hours |
|----------|----------|----------|----------|

[Figure 11B]

**Cell Migration (U87-MG, FBS 3%, SMF applied (1.0 mT), 00 Hours ~ 28 Hours)**

| 00 Hours | 04 Hours | 08 Hours | 12 Hours |
|----------|----------|----------|----------|

| 16 Hours | 20 Hours | 24 Hours | 28 Hours |
|----------|----------|----------|----------|

[Figure 12A]

**Cell Migration (U87-MG, FBS 1%, Control, 00 Hours ~ 72 Hours)**

| 00 Hours | 12 Hours | 24 Hours | 48 Hours | 72 Hours |
|---|---|---|---|---|

[Figure 12B]

**Cell Migration (U87-MG, FBS 1%, SMF applied (1.0 mT), 00 Hours ~ 72 Hours)**

| 00 Hours | 12 Hours | 24 Hours | 48 Hours | 72 Hours |
|---|---|---|---|---|

[Figure 13]

**Cell Migration (U87-MG, FBS 1%)**

[Figure 14A]

**Cell Migration (U87-MG, FBS 1%, Control, 00 Hours ~ 28 Hours)**

| 00 Hours | 04 Hours | 08 Hours | 12 Hours |
| --- | --- | --- | --- |

| 16 Hours | 20 Hours | 24 Hours | 28 Hours |
| --- | --- | --- | --- |

[Figure 14B]

**Cell Migration (U87-MG, FBS 1%, SMF applied (1.0 mT), 00 Hours ~ 28 Hours)**

| 00 Hours | 04 Hours | 08 Hours | 12 Hours |
| --- | --- | --- | --- |

| 16 Hours | 20 Hours | 24 Hours | 28 Hours |
| --- | --- | --- | --- |

[Figure 15A]

**Glioblastoma (U87-MG, Control)**

| Live | Dead | Merged |
|------|------|--------|

[Figure 15B]

**Glioblastoma (U87-MG, Control, After 24 hours)**

| Live | Dead | Merged |
|------|------|--------|

[Figure 15C]

**Glioblastoma (U87-MG, Before SMF 1.0 mT application)**

| Live | Dead | Merged |
|------|------|--------|

[Figure 15D]

**Glioblastoma (U87-MG, After SMF 1.0 mT application)**

| Live | Dead | Merged |
|------|------|--------|

[Figure 16A]

**Glioblastoma (U87-MG, Control, After 24 hours)**

| DAPI | TUNEL | Merged |
|---|---|---|

[Figure 16B]

**Glioblastoma (U87-MG, After SMF 1.0 mT application)**

| DAPI | TUNEL | Merged |
|---|---|---|

[Figure 17A]

**Glioblastoma (U87-MG, Control, After 24 hours)**

| DAPI | Ki-67 | Merged |
|---|---|---|

[Figure 17B]

**Glioblastoma (U87-MG, After SMF 1.0 mT application)**

| DAPI | Ki-67 | Merged |
|---|---|---|

[Figure 18]

[Figure 19]

Glioblastoma (U87MG)

PI3K     85 kDa

Phospho-PI3K     85 kDa

Glioblastoma (U87MG)

Akt     60 kDa

Phospho-Akt     60 kDa

[Figure 20]

Glioblastoma (U87MG)

Control     SMF applied (1.0 mT)

GPX4                   22 kDa

Glioblastoma (U87MG)

Control     SMF applied (1.0 mT)

FTH1                   21 kDa

Glioblastoma (U87MG)

Control     SMF applied (1.0 mT)

PTGS2                  80 kDa

Glioblastoma (U87MG)

Control     SMF applied (1.0 mT)

NFE2L2                 115 kDa

[Figure 21A]

U373-MG

[Figure 21B]

U373-MG+GFP

[Figure 22A]

[Figure 22B]

**Cell Proliferation (HFB-12-11-2019)**

Control
SMF applied (0.6 mT)
SMF applied (1.0 mT)

[Figure 23A]

**Cell Proliferation (U373-MG)**

Control
SMF applied (0.6 mT)

[Figure 23B]

**Cell Proliferation (U373-MG+GFP)**

Control
SMF applied (0.6 mT)

[Figure 24A]

**Cell Migration (U373-MG, FBS 5%, Control, 00 Hours ~ 28 Hours)**

| 00 Hours | 12 Hours | 24 Hours | 48 Hours | 72 Hours |
|---|---|---|---|---|

[Figure 24B]

**Cell Migration (U373-MG, FBS 5%, SMF applied (0.6 mT), 00 Hours ~ 72 Hours)**

| 00 Hours | 12 Hours | 24 Hours | 48 Hours | 72 Hours |
|---|---|---|---|---|

[Figure 25]

[Figure 26A]

**Cell Migration (U373-MG, FBS 5%, Control, 00 Hours ~ 72 Hours)**

[Figure 26B]

**Cell Migration (U373-MG, FBS 5%, SMF applied (0.6 mT), 00 Hours ~ 28 Hours)**

...

[Figure 27A]

**Cell Migration (U373-MG, FBS 3%, Control, 00 Hours ~ 72 Hours)**

| 00 Hours | 12 Hours | 24 Hours | 48 Hours | 72 Hours |

[Figure 27B]

**Cell Migration (U373-MG, FBS 3%, SMF applied (0.6 mT), 00 Hours ~ 72 Hours)**

| 00 Hours | 12 Hours | 24 Hours | 48 Hours | 72 Hours |

[Figure 28]

**Cell Migration (U373-MG, FBS 3%)**

[Figure 29A]

**Cell Migration (U373-MG, FBS 3%, Control, 00 Hours ~ 28 Hours)**

| 00 Hours | 04 Hours | 08 Hours | 12 Hours |
|---|---|---|---|

| 16 Hours | 20 Hours | 24 Hours | 28 Hours |
|---|---|---|---|

[Figure 29B]

**Cell Migration (U373-MG, FBS 3%, SMF applied (0.6 mT), 00 Hours ~ 28 Hours)**

| 00 Hours | 04 Hours | 08 Hours | 12 Hours |
|---|---|---|---|

| 16 Hours | 20 Hours | 24 Hours | 28 Hours |
|---|---|---|---|

[Figure 30A]

**Cell Migration (U373-MG, FBS 1%, Control, 00 Hours ~ 72 Hours)**

| 00 Hours | 12 Hours | 24 Hours | 48 Hours | 72 Hours |
|---|---|---|---|---|

[Figure 30B]

**Cell Migration (U373-MG, FBS 1%, SMF applied (0.6 mT), 00 Hours ~ 72 Hours)**

| 00 Hours | 12 Hours | 24 Hours | 48 Hours | 72 Hours |
|---|---|---|---|---|

[Figure 31]

[Figure 32A]

**Cell Migration (U373-MG, FBS 1%, Control, 00 Hours ~ 28 Hours)**

| 00 Hours | 04 Hours | 08 Hours | 12 Hours |
| --- | --- | --- | --- |

| 16 Hours | 20 Hours | 24 Hours | 28 Hours |
| --- | --- | --- | --- |

[Figure 32B]

**Cell Migration (U373-MG, FBS 1%, SMF applied (0.6 mT), 00 Hours ~ 28 Hours)**

| 00 Hours | 04 Hours | 08 Hours | 12 Hours |
| --- | --- | --- | --- |

| 16 Hours | 20 Hours | 24 Hours | 28 Hours |
| --- | --- | --- | --- |

[Figure 33A]

Cell Proliferation (Breast, MCF-7)

[Figure 33B]

MCF-7 (Breast, FBS 3%, Control)

MCF-7 (Breast, FBS 3%, Control)

MCF-7 (Breast, FBS 3%, Control)

[Figure 33C]

MCF-7 (Breast, FBS 3%, SMF 1.0 mT)

MCF-7 (Breast, FBS 3%, SMF 1.0 mT)

MCF-7 (Breast, FBS 3%, SMF 1.0 mT)

[Figure 34A]

Cell Proliferation (Colon, HCT-116)

[Figure 34B]

HCT-116 (Colon, FBS 3%, Control)

HCT-116 (Colon, FBS 3%, Control)

[Figure 34C]

**HCT-116 (Colon, FBS 3%, SMF 1.0 mT)**

**HCT-116 (Colon, FBS 3%, SMF 1.0 mT)**

[Figure 35A]

Cell Proliferation (Colon, HT-29)

[Figure 35B]

HT-29 (Colon, FBS 3%, Control)

HT-29 (Colon, FBS 3%, Control)

HT-29 (Colon, FBS 3%, Control)

[Figure 35C]

HT-29 (Colon, FBS 3%, SMF 1.0 mT)

[Figure 36A]

Cell Proliferation (Kidney, SNU-333)

[Figure 36B]

[Figure 36C]

**SNU-333 (Kidney, FBS 3%, SMF 1.0 mT)**

**SNU-333 (Kidney, FBS 3%, SMF 1.0 mT)**

**SNU-333 (Kidney, FBS 3%, SMF 1.0 mT)**

[Figure 37A]

**Cell Proliferation (Lung, NCI-H1299)**

[Figure 37B]

NCI-H1299 (Lung, FBS 3%, Control)

NCI-H1299 (Lung, FBS 3%, Control)

NCI-H1299 (Lung, FBS 3%, Control)

[Figure 37C]

NCI-H1299 (Lung, FBS 3%, SMF 1.0 mT)

NCI-H1299 (Lung, FBS 3%, SMF 1.0 mT)

NCI-H1299 (Lung, FBS 3%, SMF 1.0 mT)

[Figure 38A]

Cell Proliferation (Ovary, SK-OV-3)

[Figure 38B]

SK-OV-3 (Ovary, FBS 3%, Control)

SK-OV-3 (Ovary, FBS 3%, Control)

SK-OV-3 (Ovary, FBS 3%, Control)

[Figure 38C]

SK-OV-3 (Ovary, FBS 3%, SMF 1.0 mT)

SK-OV-3 (Ovary, FBS 3%, SMF 1.0 mT)

SK-OV-3 (Ovary, FBS 3%, SMF 1.0 mT)

[Figure 39A]

Cell Proliferation (Pancreas, Capan-2)

[Figure 39B]

**Capan-2 (Pancreas, FBS 3%, Control)**

**Capan-2 (Pancreas, FBS 3%, Control)**

**Capan-2 (Pancreas, FBS 3%, Control)**

[Figure 39C]

Capan-2 (Pancreas, FBS 3%, SMF 1.0 mT)

[Figure 40A]

Cell Proliferation (Prostate, DU-145)

[Figure 40B]

DU-145 (Prostate, FBS 3%, Control)

DU-145 (Prostate, FBS 3%, Control)

[Figure 40C]

**DU-145 (Prostate, FBS 3%, SMF 1.0 mT)**

**DU-145 (Prostate, FBS 3%, SMF 1.0 mT)**

[Figure 41A]

**Cell Proliferation (Prostate, PC-3)**

[Figure 41B]

PC-3 (Prostate, FBS 3%, Control)

PC-3 (Prostate, FBS 3%, Control)

PC-3 (Prostate, FBS 3%, Control)

[Figure 41C]

PC-3 (Prostate, FBS 3%, SMF 1.0 mT)

PC-3 (Prostate, FBS 3%, SMF 1.0 mT)

PC-3 (Prostate, FBS 3%, SMF 1.0 mT)

[Figure 42A]

Cell Proliferation (Skin, SK-MEL-1)

[Figure 42B]

SK-MEL-1 (Skin, FBS 3%, Control)

| Control #1 | Control #2 | Control #3 |

SK-MEL-1 (Skin, FBS 3%, Control)

| Control #4 | Control #5 | Control #6 |

SK-MEL-1 (Skin, FBS 3%, Control)

| Control #7 | Control #8 | Control #9 |

[Figure 42C]

SK-MEL-1 (Skin, FBS 3%, SMF 1.0 mT)

SK-MEL-1 (Skin, FBS 3%, SMF 1.0 mT)

SK-MEL-1 (Skin, FBS 3%, SMF 1.0 mT)

[Figure 43A]

Cell Proliferation (Stomach, NCI-N87)

[Figure 43B]

NCI-N87 (Stomach, FBS 3%, Control)

NCI-N87 (Stomach, FBS 3%, Control)

NCI-N87 (Stomach, FBS 3%, Control)

EP 4 260 901 A1

[Figure 43C]

NCI-N87 (Stomach, FBS 3%, SMF 1.0 mT)

73

[Figure 44A]

Cell Proliferation (Thyroid, SNU-790)

[Figure 44B]

SNU-790 (Thyroid, FBS 3%, Control)

SNU-790 (Thyroid, FBS 3%, Control)

SNU-790 (Thyroid, FBS 3%, Control)

[Figure 44C]

[Figure 45]

GENERATION OF
MAGNETIC FIELD

DC CURRENT

(a)

GENERATION OF
MAGNETIC FIELD

DC CURRENT

GENERATION OF
MAGNETIC FIELD

DC CURRENT

(b)

GENERATION OF UPPER AND LOWER
COIL INTERLOCKING MAGNETIC FIELD

GENERATION OF LOWER COIL
INTERLOCKING MAGNETIC FIELD

DC CURRENT

GENERATION OF UPPER AND LOWER
COIL INTERLOCKING MAGNETIC FIELD

GENERATION OF LOWER COIL
INTERLOCKING MAGNETIC FIELD
DC CURRENT

GENERATION OF UPPER AND
LOWER COIL INTERLOCKING
MAGNETIC FIELD

DC CURRENT

(c)

[Figure 46]

(a)

(b)

(c)

(d)

[Figure 47]

(a)

(b)

(c)

(d)

Europäisches
Patentamt

European
Patent Office

Office européen
des brevets

# EUROPEAN SEARCH REPORT

Application Number

EP 23 16 7312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 461 289 B1 (MUNTERMANN AXEL [DE]) 8 October 2002 (2002-10-08) * claim 6; figure 1 * | 1-16 | INV. A61N2/00 A61N2/02 |
| X | US 2006/129022 A1 (VENZA GLENN E [US] ET AL) 15 June 2006 (2006-06-15) * paragraph [0055]; claims 2,3,6; figures 7-9 * | 1-6, 11-13 | |
| X | US 2003/095022 A1 (BOYNTON THOMAS A [US] ET AL) 22 May 2003 (2003-05-22) * paragraphs [0033], [0052], [0070]; claims 1,2,9; figures 1,4 * | 1-6, 11-13 | |
| X | US 5 183 456 A (LIBOFF ABRAHAM R [US] ET AL) 2 February 1993 (1993-02-02) * column 15, lines 60-61; figures 1,6 * * column 4, lines 20-42 * | 1-6, 11-13 | |
| X | WO 97/00639 A2 (HOLCOMB ROBERT R [US]) 9 January 1997 (1997-01-09) * claim 66; figures 1,2 * | 1-6, 11-13 | TECHNICAL FIELDS SEARCHED (IPC) A61N |
| X | TINGTING LIN ET AL: "A moderate static magnetic field enhances TRAIL-induced apoptosis by the inhibition of Cdc2 and subsequent downregulation of survivin in human breast carcinoma cells", BIOELECTROMAGNETICS, JOHN WILEY, NEW YORK, NY, US, vol. 35, no. 5, 20 February 2014 (2014-02-20), pages 337-346, XP071509596, ISSN: 0197-8462, DOI: 10.1002/BEM.21849 * figure 2 * | 1-6, 11-13 | |

-/--

The present search report has been drawn up for all claims

| Place of search | Date of completion of the search | Examiner |
|---|---|---|
| Munich | 4 August 2023 | Link, Tatiana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 23 16 7312

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAMALIPOOYA SAMANEH ET AL: "Simultaneous application of cisplatin and static magnetic field enhances oxidative stress in HeLa cell line", IN VITRO CELLULAR & DEVELOPMENTAL BIOLOGY. ANIMAL, SPRINGER US, NEW YORK, vol. 53, no. 9, 21 September 2017 (2017-09-21), pages 783-790, XP036343327, ISSN: 1071-2690, DOI: 10.1007/S11626-017-0148-Z [retrieved on 2017-09-21] * abstract * ----- | 1-6, 11-13 | |

TECHNICAL FIELDS
SEARCHED     (IPC)

The present search report has been drawn up for all claims

| Place of search | Date of completion of the search | Examiner |
|---|---|---|
| Munich | 4 August 2023 | Link, Tatiana |

page 2 of 2

## ANNEX TO THE EUROPEAN SEARCH REPORT
## ON EUROPEAN PATENT APPLICATION NO.

EP 23 16 7312

This annex lists the patent family members relating to the patent documents cited in the above-mentioned European search report.
The members are as contained in the European Patent Office EDP file on
The European Patent Office is in no way liable for these particulars which are merely given for the purpose of information.

04-08-2023

| Patent document cited in search report | | Publication date | Patent family member(s) | | | Publication date |
|---|---|---|---|---|---|---|
| US 6461289 | B1 | 08-10-2002 | AT | 381955 | T | 15-01-2008 |
| | | | AU | 751973 | B2 | 05-09-2002 |
| | | | CA | 2308335 | A1 | 29-04-1999 |
| | | | CZ | 20001328 | A3 | 13-09-2000 |
| | | | DE | 29718337 | U1 | 18-02-1999 |
| | | | DE | 29824297 | U1 | 18-01-2001 |
| | | | DK | 1023106 | T3 | 13-05-2008 |
| | | | EP | 1023106 | A1 | 02-08-2000 |
| | | | EP | 1839702 | A1 | 03-10-2007 |
| | | | ES | 2296349 | T3 | 16-04-2008 |
| | | | HU | 0101370 | A2 | 28-08-2001 |
| | | | IL | 135699 | A | 27-09-2004 |
| | | | IS | 5457 | A | 17-04-2000 |
| | | | JP | 4247769 | B2 | 02-04-2009 |
| | | | JP | 2001520094 | A | 30-10-2001 |
| | | | PL | 340008 | A1 | 15-01-2001 |
| | | | PT | 1023106 | E | 16-01-2008 |
| | | | US | 6461289 | B1 | 08-10-2002 |
| | | | WO | 9920345 | A1 | 29-04-1999 |
| | | | YU | 21600 | A | 26-12-2001 |
| US 2006129022 | A1 | 15-06-2006 | EP | 1827570 | A2 | 05-09-2007 |
| | | | US | 2006129022 | A1 | 15-06-2006 |
| | | | WO | 2006060304 | A2 | 08-06-2006 |
| US 2003095022 | A1 | 22-05-2003 | NONE | | | |
| US 5183456 | A | 02-02-1993 | US | 5183456 | A | 02-02-1993 |
| | | | US | 5437600 | A | 01-08-1995 |
| WO 9700639 | A2 | 09-01-1997 | CA | 2258854 | A1 | 09-01-1997 |
| | | | NZ | 333791 | A | 29-09-2000 |
| | | | NZ | 333792 | A | 23-06-2000 |
| | | | US | 6042531 | A | 28-03-2000 |
| | | | US | 6205356 | B1 | 20-03-2001 |
| | | | US | 6280376 | B1 | 28-08-2001 |
| | | | US | 6741889 | B1 | 25-05-2004 |
| | | | WO | 9700639 | A2 | 09-01-1997 |

EPO FORM P0459

For more details about this annex : see Official Journal of the European Patent Office, No. 12/82